(19) Europäisches Patentamt
European Patent Office
Office européen des brevets

(11) EP 1 312 335 B1

(12) FASCICULE DE BREVET EUROPEEN

(45) Date de publication et mention de la délivrance du brevet:
**11.08.2010 Bulletin 2010/32**

(51) Int Cl.:
***A61Q 5/12*** *(2006.01)* ***A61K 8/898*** *(2006.01)*

(21) Numéro de dépôt: **02292781.8**

(22) Date de dépôt: **07.11.2002**

# (54) Compositions cosmétiques contenant une silicone aminée et un agent conditionneur et leurs utilisations

Kosmetische Zusammensetzungen, die Aminosilikone und Konditionierungsmittel enthalten, und deren Verwendung

Cosmetic compositions containing aminosilicone and a conditioning agent and their use

(84) Etats contractants désignés:
**AT BE BG CH CY CZ DE DK EE ES FI FR GB GR IE IT LI LU MC NL PT SE SK TR**

(30) Priorité: **08.11.2001 FR 0114476**
**08.11.2001 FR 0114477**

(43) Date de publication de la demande:
**21.05.2003 Bulletin 2003/21**

(73) Titulaire: **L'Oréal**
**75008 Paris (FR)**

(72) Inventeurs:
- **Gawtrey, Jonathan**
**92100 Boulogne (FR)**
- **Restle, Serge**
**95390 Saint-Prix (FR)**
- **Decoster, Sandrine**
**95210 Saint Gratien (FR)**
- **Lazzeri, Pascale**
**92300 Levallois Perret (FR)**

(74) Mandataire: **Le Blainvaux Bellegarde, Françoise**
**L'Oréal**
**D.I.P.I.**
**25-29 Quai Aulagnier**
**92600 Asnières (FR)**

(56) Documents cités:
**EP-A- 0 890 355 EP-A- 1 138 317**
**US-A- 4 601 902 US-A- 5 077 040**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouvelles compositions cosmétiques comprenant dans un milieu cosmétiquement acceptable au moins une silicone aminée particulière et au moins un agent conditionneur. Elle concerne également les utilisations de la silicone aminée.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe).

En outre, l'usage de silicones aminées dans ce but présente divers inconvénients. En raison de leur forte affinité pour les cheveux, certaines de ces silicones se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

En résumé, il s'avère que les compositions cosmétiques actuelles contenant des agents conditionneurs, ne donnent pas complètement satisfaction.

Le document US-A-4601902 décrit une composition cosmétique conditionnante comprenant une silicone aminée comportant au moins un groupe aminoéthyliminopropyl et des agents conditionneurs conventionnels.

La demanderesse a maintenant découvert que l'association d'une silicone aminée particulière avec des agents conditionneurs permet de remédier à ces inconvénients.

Ainsi, à la suite d'importantes recherches menées sur la question, il a maintenant été trouvé par la Demanderesse qu'une composition comprenant au moins une silicone aminée telle que définie ci-dessous et au moins un agent conditionneur, permettait de limiter, voire supprimer, le manque de brillance, de lissage et de douceur, des cheveux, tout en conservant les autres propriétés cosmétiques avantageuses qui sont attachées aux compositions contenant une silicone.

Cette association apporte des propriétés cosmétiques améliorées (légèreté, démêlage, volume, lissage, brillance), et de plus les effets sont persistants et rémanents.

La demanderesse a ainsi découvert que la rémanence des propriétés cosmétiques ( en particulier les effets de conditionnement) étaient améliorées par la présence de la silicone aminée particulière.

Par ailleurs, les compositions de l'invention appliquées sur la peau notamment sous forme de bain moussant ou de gel douche, apportent une amélioration de la douceur de la peau.

Les compositions selon l'invention favorisent le dépôt sur les matières kératiniques des agents conditionneurs.

Ainsi, selon la présente invention, il est maintenant proposé de nouvelles compositions cosmétiques, comprenant, dans un milieu cosmétiquement acceptable, au moins une silicone aminée telle que définie ci-dessous et au moins un agent conditionneur.

Un autre objet de l'invention concerne l'utilisation d'au moins une silicone aminée telle que définie ci-dessous dans, ou pour la fabrication d'une composition cosmétique comprenant un agent conditionneur.

Un autre objet de l'invention concerne l'utilisation d'une composition comprenant au moins une silicone aminée telle que définie ci-dessous et un agent conditionneur pour le conditionnement des matières kératiniques.

Un autre objet de l'invention concerne l'utilisation d'une composition comprenant au moins une silicone aminée telle que définie ci-dessous et au moins un agent conditionneur pour le conditionnement des matières kératiniques.

Un autre objet de l'invention concerne l'utilisation d'une composition comprenant au moins une silicone aminée telle que définie ci-dessous et au moins un agent conditionneur pour améliorer la légèreté, la douceur, la brillance et/ou le démêlage, pour faciliter le coiffage des matières kératiniques.

Un autre objet de l'invention concerne l'utilisation d'une composition comprenant au moins une silicone aminée telle que définie ci-dessous et au moins un agent conditionneur pour améliorer la rémanence des effets du conditionnement aux shampooings.

Un autre objet de l'invention concerne l'utilisation d'au moins une silicone aminée telle que définie ci-dessous pour améliorer la rémanence des effets du conditionnement d'une composition cosmétique aux shampooings.

Un autre objet de l'invention concerne également le procédé pour améliorer la rémanence des effets du conditionnement d'une composition cosmétique aux shampooings qui consiste à introduire la silicone aminée définie ci-dessous dans une composition cosmétique.

Les différents objets de l'invention vont maintenant être détaillés. L'ensemble des significations et définitions

des composés utilisés dans la présente invention données ci-dessous sont valables pour l'ensemble des objets de l'invention.

Selon la présente invention, par matières kératiniques, on comprend les cheveux, les cils, les sourcils, la peau, les ongles, les muqueuses ou le cuir chevelu et plus particulièrement les cheveux.

Les silicones aminées selon l'invention sont choisies parmi celles de formules (I) ou (II) suivantes :

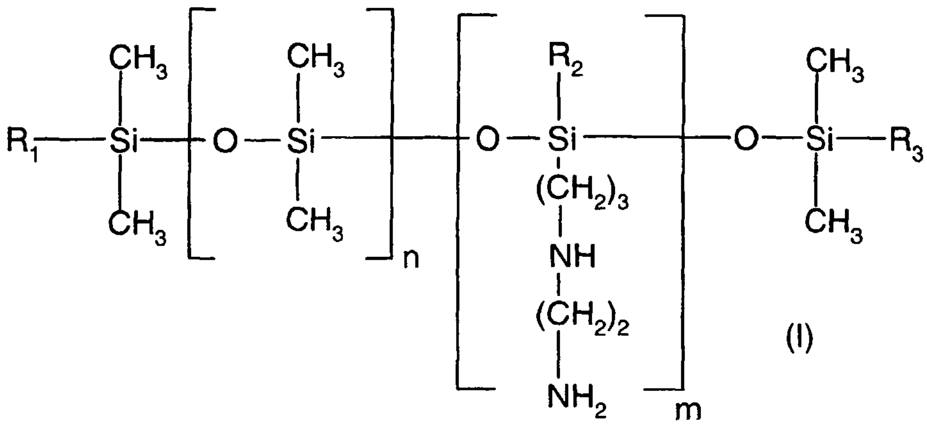

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;

$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.
De préférence le radical alcoxy est un radical méthoxy.
Le rapport molaire Hydroxy/Alcoxy va de préférence de 0,2:1 à 0,4:1 et de préférence de 0,25:1 à 0,35:1 et plus particulièrement est égal à 0,3:1.

La masse moléculaire moyenne en poids de la silicone va de préférence comprise de 2.000 à 1.000.000 et encore plus particulièrement de 3.500 à 200.000.

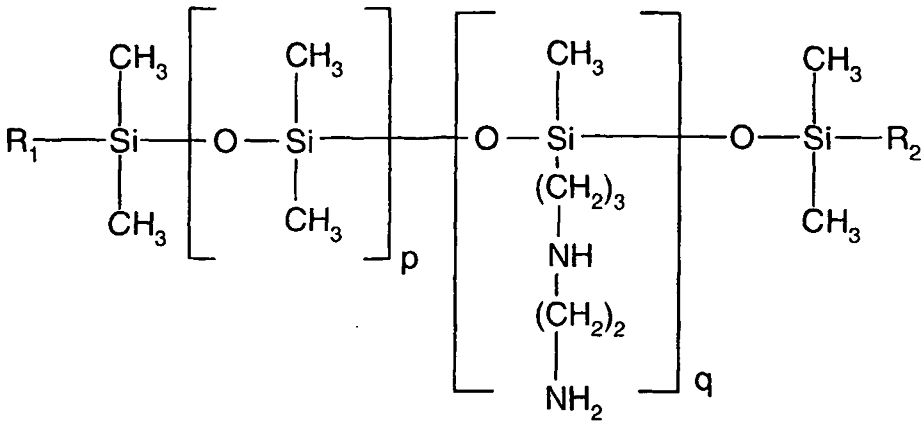

(II)

dans laquelle :

p et q sont des nombres tels que la somme (p + q) peut varier notamment de 1 à 1 000 et en particulier de 50 à 350, et plus particulièrement de 150 à 250,

p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$, $R_2$, différents, représentent un radical hydroxy ou alcoxy en $C_1$-$C_4$, l'un au moins des radicaux $R_1$ ou $R_2$ désignant un radical alcoxy.

De préférence le radical alcoxy est un radical méthoxy.

Le rapport molaire Hydroxy/Alcoxy va généralement de 1:0,8 à 1:1,1 et de préférence de 1:0,9 à 1:1 et plus particulièrement est égal à 1:0,95.

La masse moléculaire moyenne en poids de la silicone va de préférence de 2.000 à 200.000 et encore plus particulièrement de 5.000 à 100.000 et plus particulièrement de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des formules (I) et (II).

Un produit contenant des silicones aminées de structure (I) est proposé par la société WACKER sous la dénomination BELSIL® ADM 652.

Un produit contenant des silicones aminées de structure (II) est proposé par WACKER sous la dénomination Fluid WR 1300®.

Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationique et/ou non ionique.

La taille moyenne en nombre des particules de silicone dans l'émulsion est généralement comprise entre 3 nm et 500 nanomètres .

De préférence, notamment comme silicones aminées de formule (II), on utilise des microémulsions dont la taille moyenne des particule est comprise entre 5 nm et 60 nanomètres (bornes incluses) et plus particulièrement entre 10 nm et 50 nanomètres (bornes incluses).

Ainsi, on peut utiliser selon l'invention les microémulsions de silicone aminée de formule (II) proposées sous les dénominations FINISH CT 96 E® ou SLM 28020® par la société WACKER.

De préférence la silicone aminée est choisie de façon telle que l'angle de contact avec l'eau d'un cheveu traité avec une composition contenant 2% (matière active) de ladite silicone selon l'invention soit compris entre 90° et 180° (bornes incluses) et de préférence compris entre 90 et 130° (bornes incluses).

Pour mesurer l'angle de contact, la silicone aminée est de préférence solubilisée
ou dispersée dans un solvant de la silicone aminée ou de l'émulsion de silicone aminée (notamment l'hexaméthyldisiloxane ou l'eau selon l'hydrophilie de la silicone).

De préférence la composition contenant la ou les silicones aminées de formule (I) ou (II) est telle que l'angle de contact avec l'eau d'un cheveu traité avec ladite composition est compris entre 90 et 180°(bornes incluses) et de préférence entre 90 et 130° (bornes incluses).

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90° (borne excluse), hydrophobe lorsque cet angle est compris entre 90° et 180° (bornes incluses).

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée à l'eau distillée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique.

La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et $\theta$ l'angle de contact.

Le produit SLM 28020 de WACKER à 12% dans l'eau (soit 2% en silicone aminée) conduit à un angle de contact de 93° selon le test indiqué ci-dessus.

Le produit BELSIL ADM 652 de WACKER à 2% dans l'hexaméthyldisiloxane (soit 2% en silicone aminée) conduit à un angle de contact de 111° selon le test indiqué ci-dessus.

La ou les silicones aminées de formule (I) ou (II) sont utilisées de préférence en une quantité comprise allant de 0,01 à 20% en poids par rapport au poids total de la composition. Plus préférentiellement, cette quantité va de 0,1 à 15% en poids et encore plus particulièrement de 0,5 à 10 % en poids par rapport au poids total de la composition.

Dans le cadre de la présente demande, on entend par agent conditionneur tout agent ayant pour fonction l'amélioration des propriétés cosmétiques des matières kératiniques telles que les cheveux, en particulier la douceur, le démêlage, le toucher, le lissage, l'électricité statique.

Les agents conditionneurs peuvent être solubles ou insolubles dans l'eau.

Les agents conditionneurs sont notamment choisis parmi les huiles de synthèses telles que les poly-oléfines, les huiles minérales, les huiles végétales, les huiles fluorées ou perfluorées, les cires naturelles ou synthétiques, les composés de type céramide, les esters d'acides carboxyliques, les silicones différentes de celles de formules (I) ou (II), parmi les polymères anioniques, les polymères non ioniques, les polymères cationiques, les polymères amphotères, les protéines cationiques, les hydrolysats de protéines cationiques et les tensioactifs cationiques , habituellement utilisés dans les compositions cosmétiques ou dermatologiques ainsi que les mélanges de ces différents composés.

Les agents conditionneurs conformément à l'invention peuvent être solides, liquides ou pâteux à température ambiante (25°C) et à pression atmosphérique, ils peuvent notamment se présenter sous forme de poudre, d'huiles, de cires, de résines ou de gommes.

Les agents conditionneurs insolubles sont notamment dispersés dans les compositions sous forme de particules ayant généralement une taille moyenne en nombre comprise entre 2 nanomètres et 100 microns, de préférence entre 30 nanomètres et 20 microns (mesurée avec un granulomètre).

Les agents conditionneurs insolubles dans l'eau sont insolubles dans l'eau à une concentration supérieure ou égale à 0,1% en poids dans l'eau à 25°C, c'est à dire qu'ils ne forment pas dans ces conditions une solution macroscopiquement isotrope transparente.

Les huiles de synthèse sont notamment les polyoléfines en particulier les poly-$\alpha$-oléfines et plus particulièrement :

- de type polybutène, hydrogéné ou non, et de préférence polyisobutène, hydrogéné ou non.
  On utilise de préférence les oligomères d'isobutylène de poids moléculaire inférieur à 1000 et leurs mélange avec des polyisobutylènes de poids moléculaire supérieur à 1000 et de préférence allant de 1000 et 15000.
  A titre d'exemples de poly-$\alpha$-oléfines utilisables dans le cadre de la présente invention, on peut plus particulièrement mentionner les polyisobutènes vendus sous le nom de PERMETHYL® 99 A, 101 A , 102 A , 104 A (n=16) et 106 A (n=38) par la Société PRESPERSE Inc, ou bien encore les produits vendus sous le nom de ARLAMOL® HD (n=3) par la Société ICI (n désignant le degré de polymérisation),
- de type polydécène, hydrogéné ou non.

De tels produits sont vendus par exemple sous les dénominations ETHYLFLO® par la société ETHYL CORP., et d'ARLAMOL® PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule $R_9COOR_{10}$ dans laquelle $R_9$ représente le reste d'un acide gras supérieur comportant de 7 à 29 atomes de carbone et $R_{10}$ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;
On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre

1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Selon la présente invention, les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine

ou les mélanges de ces composés.

Les huiles fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Les alcools gras peuvent être choisis parmi les alcools gras en $C_8$-$C_{22}$, linéaires ou ramifiés ; ils sont éventuellement oxyalkylénés avec 1 à 15 moles d'oxyde d'alkylène ou polyglycérolés avec 1 à 6 moles de glycérol. L'oxyde d'alkylène est de préférence l'oxyde d'éthylène et/ou de propylène.

Les esters d'acides carboxyliques sont en particulier les esters mono, di, tri ou tétracarboxyliques.

Les esters d'acides monocarboxyliques sont notamment les monoesters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en $C_{12}$-$C_{15}$; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Les silicones différentes de celles de formules (I) ou (II) utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.

On peut également citer les cyclocopolymères du type diméthylsiloxanes/méthylakylsiloxane, tel que la "SILICONE VOLATILE FZ 3109" commercialisée par la société UNION CARBIDE, de structure chimique : avec

avec D :
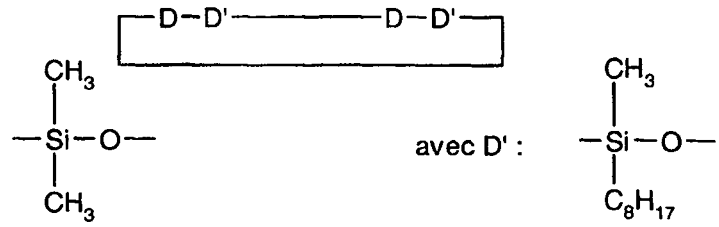
avec D' :

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$.

La viscosité des silicones est par exemple mesurée à 25°C selon la norme ASTM 445 Appendice C.

Parmi ces polyalkylsiloxanes, on peut citer à titre non limitatif les produits commerciaux suivants :

- les huiles SILBIONE® des séries 47 et 70 047 ou les huiles MIRASIL® commercialisées par RHODIA CHIMIE telles que par exemple l'huile 70 047 V 500 000;
- les huiles de la série MIRASIL commercialisées par la société RHODIA CHIMIE ;
- les huiles de la série 200 de la société DOW CORNING telles que plus particulièrement la DC200 de viscosité 60

000 cSt ($mm^2/s$);

- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL® WAX 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2} m^2/s$ à 25°C.

Parmi ces polyalkylarylsiloxanes on peut citer à titre d'exemple les produits commercialisés sous les dénominations suivantes :

. les huiles SILBIONE de la série 70 641 de RHODIA CHIMIE ;
. les huiles des séries RHODORSIL 70 633 et 763 de RHODIA CHIMIE ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydlméthylsiloxanes/méthylvlnylslloxane,
- polydiméthylslloxane/diphénylslloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies

précédemment et comportant dans leur structure un ou plusieurs groupements organofonctionnels fixés par l'intermédiaire d'un radical hydrocarboné.

Parmi les silicones organomodifiées différentes de celles de formules (I) ou (II), on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 et l'alkyl ($C_{12}$) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334
- des groupements acyloxyalkyle tels que par exemple les polyorganosiloxanes décrits dans le brevet US-A-4957732 .
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

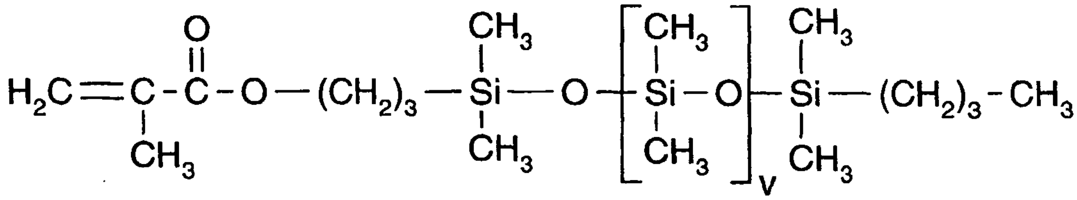

(II)

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les silicones particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 cSt, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones autres que celle de formule (I) ou les triméthylsilylamodiméthicone.

Les agents conditionneurs qui sont cités ci-dessous sont généralement solubles dans l'eau en particulier les polymères anioniques, les polymères non ioniques, les polymères cationiques, les polymères amphotères, les protéines cationiques, les hydrolysats de protéines cationiques et les tensioactifs cationiques ainsi que les mélanges de ces différents composés.

Les polymères anioniques généralement utilisés sont des polymères comportant des groupements dérivés d'acide carboxylique, sulfonique ou phosphorique et ayant de préférence un poids moléculaire moyen en poids compris entre environ 500 et 5.000.000 déterminé par exemple par chromatographie par perméation de gel.

Les polymères anionique peuvent être choisis parmi :

1) les polymères comportant des motifs carboxyliques dérivant de monomères mono ou diacides carboxyliques insaturés tels que ceux répondant à la formule :

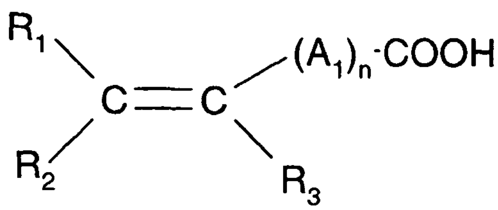

(III)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, $R_1$ désigne un atome d'hydrogène, un groupement phényle ou benzyle, $R_2$ désigne un atome d'hydrogène, un groupement alkyle inférieur ou carboxyle, $R_3$ désigne un atome d'hydrogène, un groupement alkyle inférieur, un groupement $-CH_2-COOH$, phényle ou benzyle ; Dans la formule précitée un radical alkyle inférieur désigne de préférence un groupement ayant 1 à 4 atomes de carbone et en particulier, méthyle et éthyle.

Les polymères filmogènes anioniques à groupements carboxyliques préférés selon l'invention sont :

A) Les homo- ou copolymères d'acide acrylique ou méthacrylique ou leurs sels et en particulier les produits vendus sous les dénominations VERSICOL E ou K par la société CIBA et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.

B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en $C_1$-$C_4$ et les terpolymères de vinylpyrrolidone, d'acide acrylique

et de méthacrylate d'alkyle en $C_1$-$C_{20}$ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.

C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique $\alpha$- ou $\beta$-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.

D) les copolymères dérivés d'acides ou d'anhydrides carboxyliques monoinsaturés en $C_4$-$C_8$ choisis parmi :

- les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.

- les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, $\alpha$-oléfine, esters acryliques ou méthacryliques, acides acrylique ou méthacrylique ou vinylpyrrolidone dans leur chaîne,
  les fonctions anhydrides de ces copolymères étant éventuellement monoestérifiées ou monoamidifiées.
  Ces polymères sont par exemple décrits dans les brevets français 2.350.384 et 2.357.241 de la demanderesse.

E) les polyacrylamides comportant des groupements carboxylates.

2) Les polymères comprenant les groupements sulfoniques sont des polymères comportant des motifs vinylsulfonique, styrène sulfonique, naphtalène sulfonique ou acrylamido alkylsulfonique.
Ces polymères peuvent être notamment choisis parmi :

- les sels de l'acide polyvinylsulfonique ayant un poids moléculaire moyen en poids compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.

- les sels de l'acide polystyrène sulfonique les sels de sodium ayant un poids moléculaire moyen en poids d'environ 500.000 et d'environ 100.000 vendus respectivement sous les dénominations Flexan 500 et Flexan 130 par National Starch. Ces composés sont décrits dans le brevet FR 2.198.719.

- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par COGNIS.

Selon l'invention, les polymères notamment filmogènes anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous

la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères filmogènes anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes; K et M peuvent également désigner une chaîne polymère cationique comportant des groupements aminé primaire, secondaire, tertiaire ou quaternaire, dans laquelle au moins l'un des groupements aminé porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société COGNIS.
Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société NALCO.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acryiamide, le N-octylacrylamide, le N-décylacrylamide, le N-dodécylacrylamide ainsi que les méthacrylamides correspondants.
Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
Les comonomères basiques préférés sont des méthacrylates d'aminoéthyle, de butyl aminoéthyle, de N,N'-diméthylaminoéthyle, de N-tertio-butylaminoéthyle.
On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale:

$$[CO-R_4-CO-Z] \qquad (IV)$$

dans laquelle $R_4$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

$$-NH-[(CH_2)_x-NH]_p- \qquad (V)$$

où x=2 et p=2 ou 3, ou bien x=3 et p=2
ce radical dérivant de la diéthylène triamine, de la triéthylène tétraamine ou de la dipropylène triamine;
b) dans les proportions de 0 à 40 moles % le radical (V) ci-dessus, dans lequel x=2 et p=1 et qui dérive de l'éthylènediamine, ou le radical dérivant de la pipérazine :

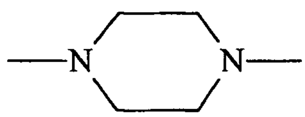

c) dans les proportions de 0 à 20 moles % le radical -NH-$(CH_2)_6$-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple lès acides acrylique, méthacrylique, itaconique.
Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule :

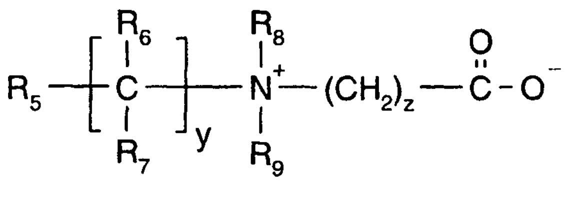
(VI)

dans laquelle $R_5$ désigne un groupement insaturé polymérisable tel qu'un groupement acrylate, méthacrylate, acrylamide ou méthacrylamide, y et z représentent un nombre entier de 1 à 3, $R_6$ et $R_7$ représentent un atome d'hydrogène, méthyle, éthyle ou propyle, $R_8$ et $R_9$ représentent un atome d'hydrogène ou un radical alkyle de telle façon que la somme des atomes de carbone dans $R_8$ et $R_9$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de butyle/méthacrylate de diméthyl-carboxyméthylammonio-éthyl tel que le produit vendu sous la dénomination DIAFORMER Z301 par la société SANDOZ.

(5) les polymères dérivés du chitosane comportant des motifs monomères répondant aux formules (VII), (VIII), (IX) suivantes :

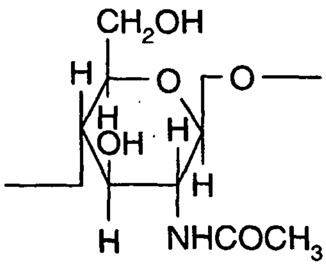

(VII)

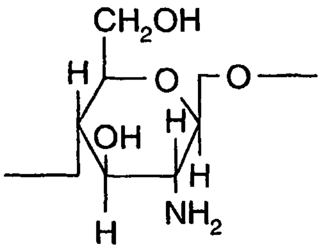

(VIII)

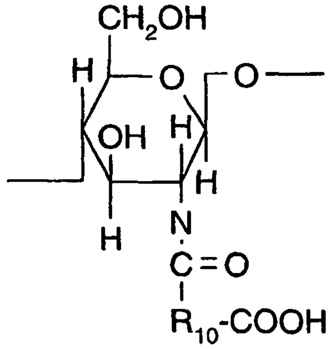

(IX)

le motif (VII) étant présent dans des proportions comprises entre 0 et 30%, le motif (VIII) dans des proportions comprises entre 5 et 50% et le motif (IX) dans des proportions comprises entre 30 et 90%, étant entendu que dans ce motif (IX), $R_{10}$ représente un radical de formule:

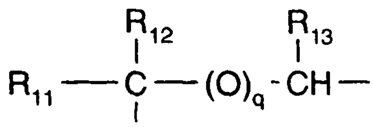

dans laquelle désigne zéro ou 1
si q=0, $R_{11}$, $R_{12}$ et $R_{13}$, identiques ou différents, représentent chacun un atome d'hydrogène, un radical méthyle, hydroxyle, acétoxy ou amino, un radical monoalcoylamine ou un radical dialcoylamine éventuellement interrompus par un
ou plusieurs atomes d'azote et/ou éventuellement substitués par un ou plusieurs groupes amine, hydroxyle, carboxyle, alcoylthio, sulfonique, un radical alcoylthio dont le groupe alcoyle porte un groupe amino, l'un au moins des radicaux $R_{11}$, $R_{12}$ et $R_{13}$ étant dans ce cas un atome d'hydrogène ;
ou si q=1, $R_{11}$, $R_{12}$ et $R_{13}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (X) tels que ceux décrits par exemple dans le brevet français 1 400 366 :

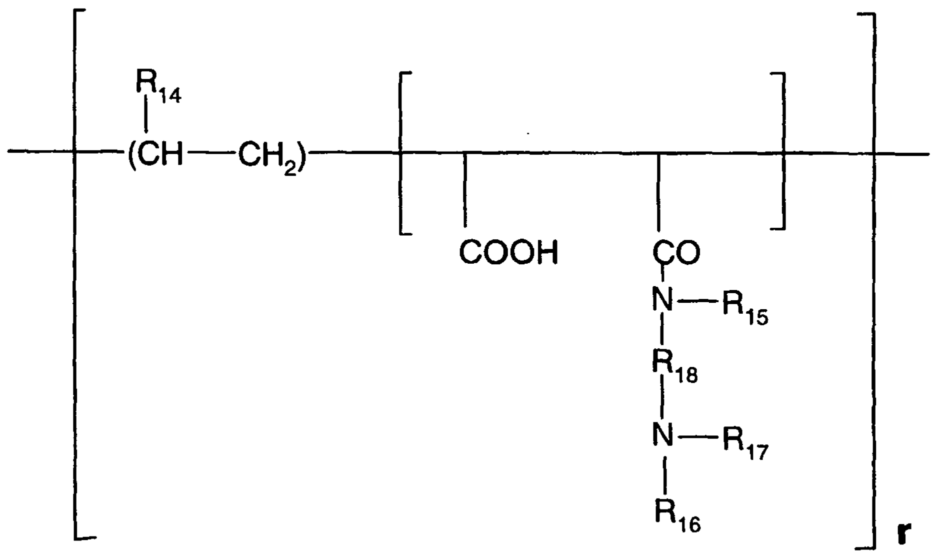

(X)

dans laquelle $R_{14}$ représente un atome d'hydrogène, un radical $CH_3O$, $CH_3CH_2O$, phényle, $R_{15}$ désigne l'hydrogène ou un radical alkyle inférieur tel que méthyle, éthyle, $R_{16}$ désigne l'hydrogène ou un radical alkyle inférieur tel que méthyle, éthyle, $R_{17}$ désigne un radical alkyle inférieur tel que méthyle, éthyle ou un radical répondant à la formule : $-R_{18}-N(R-_{16})_2$, $R_{18}$ représentant un groupement $-CH_2-CH_2-$, $-CH_2-CH_2-CH_2-$, $-CH_2-CH(CH_3)-$, $R_{16}$ ayant les significations mentionnées ci-dessus,
ainsi que les homologues supérieurs de ces radicaux et contenant jusqu'à 6 atomes de carbone,
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

- D-X-D-X-D- (XI)

où D désigne un radical

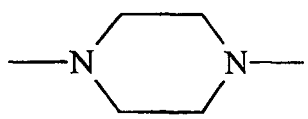

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'aminé, ammonium quaternaire, amide, imide, alcool, ester et/ou uréthanne ;

b) les polymères de formule :

-D-X-D-X- (XII)

où D désigne un radical

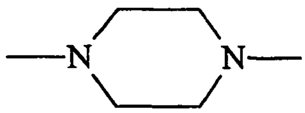

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Les polymères amphotères particulièrement préférés selon l'invention sont ceux de la famille (1).
Les polymères non ioniques utilisables selon la présente invention sont choisis par exemple parmi :

- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHODIA CHIMIE ;
- les copolymères d'acétate de vinyle et d'ester acrylique tels que le produit proposé sous le nom de RHODOPAS AD 310 de RHODIA CHIMIE ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth) acrylates d'alkyle ; on peut citer les produits proposés sous les dénominations NIPOL LX 531 B par la société NIPPON ZEON

ou ceux proposés sous la dénomination CJ 0601 B par la société ROHM & HAAS ;

- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHODIA CHIMIE.
- les gommes de guar non ioniques chimiquement modifiées ou non modifiées. Les gommes de guar non ioniques non modifiées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupe-

ments hydroxyalkyle en $C_1$-$C_6$. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

On peut aussi utiliser, comme polymères, des polyuréthannes fonctionnalisés ou non.

Les polyuréthannes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements aminé primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

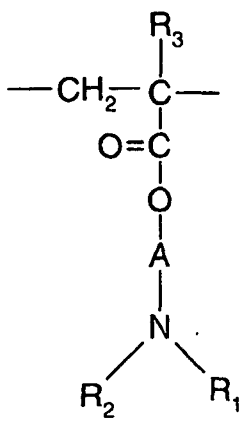 (XIII),

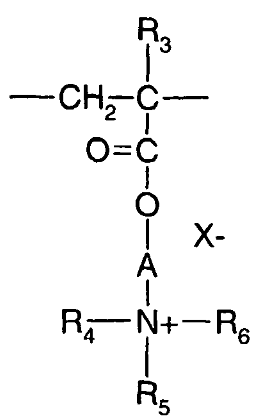 (XIV)

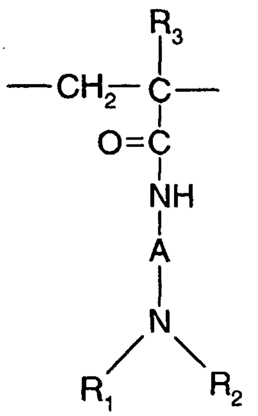 (XV)

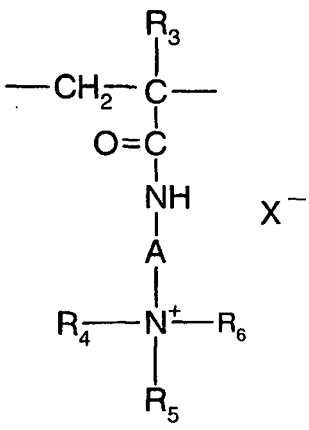 (XVI)

dans lesquelles:

- $R_1$ et $R_2$, identiques ou différents, représentent hydrogène ou un groupe alkyle ayant de 1 à 6 atomes de carbone et de préférence méthyle ou éthyle;
- $R_3$, identiques ou différents, désignent un atome d'hydrogène ou un radical $CH_3$;
- A, identiques où différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
- $R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
- X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA ,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT® 734" ou "GAFQUAT® 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX® VC 713 par la société ISP,
- les copolymères vinylpyrrolidone / méthacrylamidopropyl dimethylamine commercialisés notamment sous la dénomination STYLEZE® CC 10 par ISP.
- et les copolymères vinylpyrrolidone / méthacrylamide de diméthylaminopropyle quaternisé tel que le produit vendu sous la dénomination "GAFQUAT® HS 100" par la société ISP.

2) Les polysaccharides cationiques notamment les celluloses et les gommes de galactomannanes cationiques. Parmi les polysaccharides cationiques, on peut citer plus particulièrement. les dérivés d'éthers de cellulose comportant des groupements ammonium quaternaires, les copolymères de cellulose cationiques
ou les dérivés de cellulose greffés avec un monomère hydrosoluble d'ammonium quaternaire et les gommes de galactomannanes cationiques.

Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la société AMERCHOL. Ces polymères sont également définis dans le dictionnaire CTFA comme des ammonium quaternaires d'hydroxyéthylcellulose ayant réagi avec un époxyde substitué par un groupement triméthylammonium.
Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "CELQUAT® L 200" et "CELQUAT® H 100" par la société NATIONAL STARCH.
Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR® C13S, JAGUAR® C 15, JAGUAR® C 17 ou JAGUAR® C162 par la société MEYHALL.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "CARTARETINE® F, F4 ou F8" par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "HERCOSETT® 57" par la société HERCULES Inc. ou bien sous la dénomination de "PD 170" ou "DELSETTE® 101" par la société HERCULES dans le cas du copolymère d'acide adipique/époxypropyl/diéthylène-triamine.

(7) les cyclopolymères d'alkyl diallyl amine ou de dialkyl diallyl ammonium tels que les homopolymères ou copolymères comportant comme constituant principal de la chaîne des motifs répondant aux formules (XVII) ou (XVIII) :

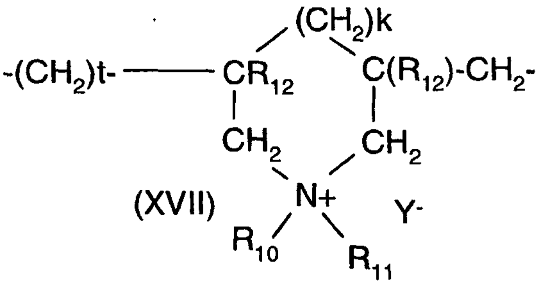

(XVII)

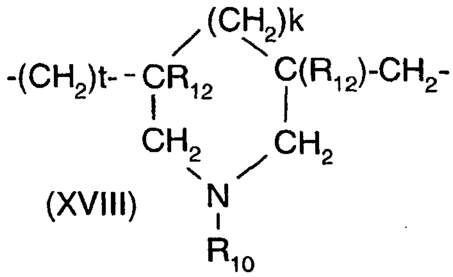

(XVIII)

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. $R_{10}$ et $R_{11}$, indépendamment l'un de l'autre, désignent de préférence un groupement alkyle ayant de 1 à 4 atomes de carbone.
Parmi les polymères définis ci-dessus, on peut citer plus particulièrement l'homopolymère de chlorure de diméthyl-diallylammonium vendu sous la dénomination "MERQUAT® 100" par la société NALCO (et ses homologues de faibles masses moléculaires moyenne en poids) et les copolymères de chlorure de diallyldiméthylammonium et d'acrylamide commercialisés sous la dénomination "MERQUAT® 550".

(8) le polymère de diammonium quaternaire contenant des motifs récurrents répondant à la formule :

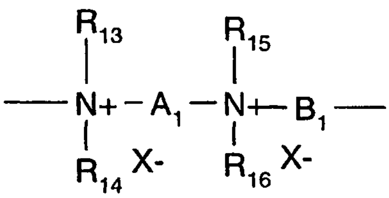

(XIX)

formule (XIX) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n- dans lequel n est compris entre 1 et 100 et de préférence entre 1 et 50, et
dans lequel D désigne :

a) un reste de glycol de formule : -O-Z-O-, où Z désigne un radical hydrocarboné linéaire ou ramifié ou un

groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule :

-NH-CO-NH- ;

De préférence, $X^-$ est un anion tel que le chlorure ou le bromure.
Ces polymères ont une masse moléculaire moyenne en nombre généralement comprise généralement entre 1.000 et 100.000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule :

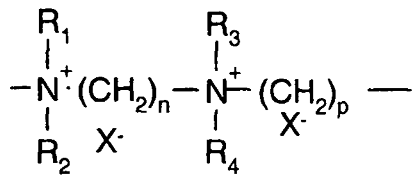
(XX)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.
Un composé de formule (XX) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(9) les polymères de polyammonium quaternaires constitués de motifs de formule (XXI):

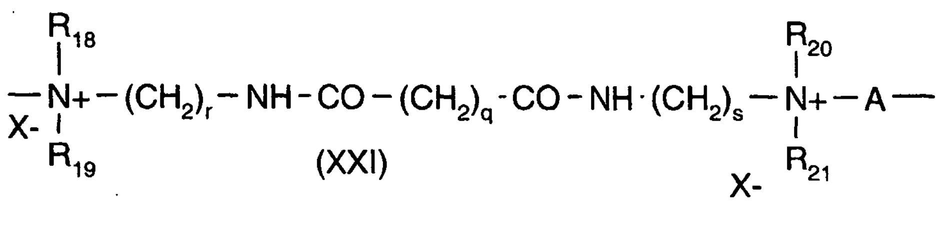
(XXI)

ormule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,

où p est égal à 0 ou à un nombre entier allant de 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers allant de 1 et 6,
q est égal à 0 ou à un nombre entier allant de 1 et 34,
X- désigne un anion tel qu'un halogènure,
A désigne un radical divalent et représente de préférence $-CH_2-CH_2-O-CH_2-CH2-$.

De tels composés sont notamment décrits dans la demande de brevet EP-A-122 324.
On peut par exemple citer parmi ceux-ci, les produits "MIRAPOL® A 15", "MIRAPOL® AD1", "MIRAPOL® AZ1" et "MIRAPOL® 175" vendus par la société MIRANOL.

(10) Les polymères quaternaires de vinylpyrrolidone et de vinylimidazole tels que par exemple les produits commercialisés sous les dénominations LUVIQUAT® FC 905, FC 550 et FC 370 par la société B.A.S.F.

(11) Les polyamines comme le POLYQUART® H vendu par COGNIS, référencé sous le nom de « POLYETHYLENEGLYCOL (15) TALLOW POLYAMINE » dans le dictionnaire CTFA.

(12) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la société AMERCHOL, les cyclopolymères cationiques, en particulier les homopolymères ou copolymères de chlorure de diméthyldiallylammonium, vendus sous les dénominations « MERQUAT® 100 », «MERQUAT® 550 » et « MERQUAT® S » par la société NALCO, les polymères quaternaires de vinylpyrrolidone et de vinylimidazole et leurs mélanges.

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :

- les hydrolysats de collagène portant des groupements triéthylammonium tels que les produits vendus sous la dénomination "Quat-Pro E" par la société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "CROTEIN® BTA" par la société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :

- le "CROQUAT® L" dont les groupements ammonium quaternaires comportent un groupement alkyle en $C_{12}$ ;
- le "CROQUAT® M" dont les groupements ammonium quaternaires comportent des groupements alkyle en $C_{10}$-$C_{18}$;
- le "CROQUAT® S" dont les groupements ammonium quaternaires comportent un groupement alkyle en $C_{18}$ ;
- le "CROTEIN® Q" dont les groupements ammonium quaternaires comportent au moins un groupe alkyle ayant de 1 à 18 atomes de carbone.

Ces différents produits sont vendus par la société CRODA.

D'autres protéines ou hydrolysats quaternisés sont par exemple ceux répondant à la formule :

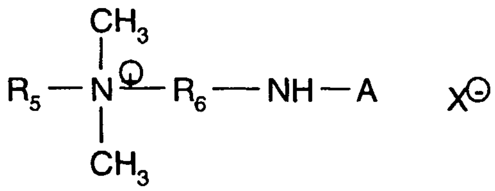

(XXII)

dans laquelle X⁻ est un anion d'un acide organique ou minéral, A désigne un reste de protéine dérivé d'hydrolysats de protéine notamment de collagène, $R_5$ désigne un groupement lipophile comportant jusqu'à 30 atomes de carbone, $R_6$ représente un groupement alkylène ayant 1 à 6 atomes de carbone. On peut citer par exemple les produits vendus par la société INOLEX, sous la dénomination "LEXEIN® QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société CRODA sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les tensioactifs cationiques sont notamment choisis parmi les sels d'ammonium quaternaires, les sels d'ammonium quaternaires de l'imidazoline, les sels de diammonium quaternaires, les sels d'ammonium quaternaire contenant au moins une fonction ester.

Les tensioactifs cationiques peuvent être choisis parmi :

A) les sels d'ammonium quaternaires de la formule générale (XXIII) suivante :

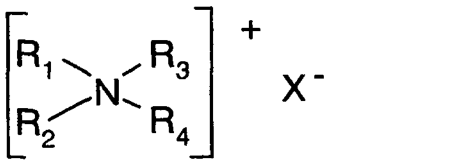

(XXIII)

dans laquelle X- est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate.
, et

i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide, R4 désigne un radical alkyle, linéaire ou ramifié, comportant de 16 à 30 atomes de carbone.
De préférence le tensioactif cationique est un sel (par exemple chlorure) de béhényl triméthyl ammonium.

ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide

et hydroxyalkyle, comportant environ de 1 à 4 atomes de carbone;
R3 et R4, identiques ou différents, désignent un radical alkyle, linéaire ou ramifié, comportant de 12 à 30 atomes de carbone, ledit radical comprenant au moins une fonction ester ou amide.
R3 et R4 sont notamment choisis parmi les radicaux alkyl($C_{12}$-$C_{22}$)amido alkyle($C_2$-$C_6$), alkyl($C_{12}$-$C_{22}$)acétate ;
De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (XXIV) suivante :

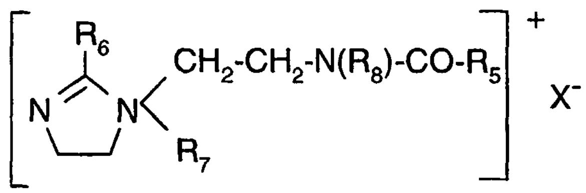

(XXIV)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$, $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,

C) - les sels de diammonium quaternaire de formule (XXV) :

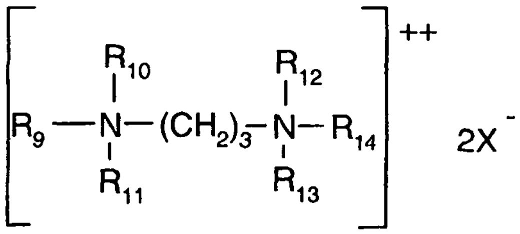

(XXV)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (XXVI) suivante :

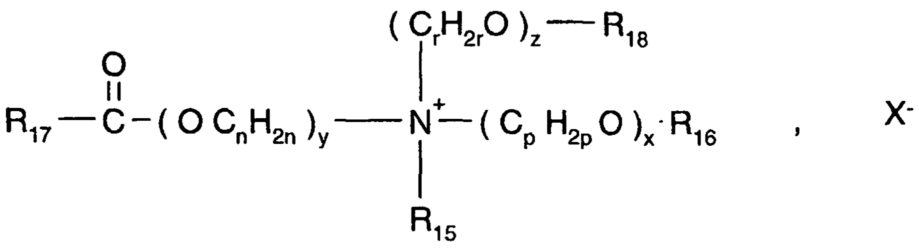

(XXVI)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical

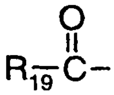

  - les radicaux $R_{20}$ hydrocarbonés en $C_1$-$C_{22}$ linéaires ou ramifiés, saturés ou insaturés,
  - l'atome d'hydrogène,
- $R_{18}$ est choisi parmi :
  - le radical

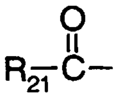

  - les radicaux $R_{22}$ hydrocarbonés en $C_1$-$C_6$ linéaires ou ramifiés, saturés ou insaturés,
  - l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors R16 désigne R20 et que lorsque z vaut 0 alors R18 désigne R22.

On utilise plus particulièrement les sels d'ammonium de formule (XXVI) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :
  - le radical

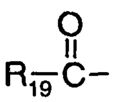

  - les radicaux méthyle, éthyle ou hydrocarbonés en $C_{14}$-$C_{22}$
  - l'atome d'hydrogène ;
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;

- $R_{18}$ est choisi parmi :

  - le radical

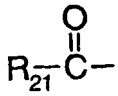

  - l'atome d'hydrogène ;

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-WITCO.

Parmi les sels d'ammonium quaternaire on préfère le chlorure de stéaramidopropyldiméthyl (myristyl acétate) ammonium commercialisé sous la dénomination «CERAPHYL® 70» par la société VAN DYK, le Quaternium-27 ou le Quaternium-83 commercialisés par la société WITCO.

Les tensioactifs cationiques sont classés dans la classe des agents conditionneurs solubles mais selon leurs structures chimiques, certains tensioactifs cationiques peuvent être insolubles dans l'eau.

Selon l'invention, la ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Selon un mode de réalisation particulièrement préféré, les compositions selon l'invention comprennent au moins un polymère cationique et/ou au moins une silicone .

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

(i) <u>Tensioactif(s) anionique(s) :</u>

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) <u>Tensioactif(s) non ionique(s)</u> :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés ou les acides

gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{-CONHCH}_2\text{CH}_2\text{-N}^+(R_3)(R_4)(\text{CH}_2\text{COO-}) \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_5\text{-CONHCH}_2\text{CH}_2\text{-N(B)(C)} \qquad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$- CHOH - SO3H
$R_5$ désigne un radical alkyle d'un acide $R_5$ -COOH présent dans l'huile de coprah ou de l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, Sème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.

(iv) Tensioactif(s) cationique(s) :

Les tensioactifs cationiques peuvent être choisis parmi :

A) les sels d'ammonium quaternaires de la formule générale (IX) suivante :

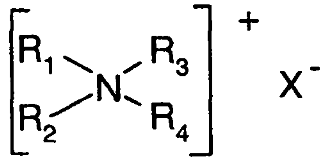

(IX)

dans laquelle $X^-$ est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate. , et

i) les radicaux $R_1$ à $R_3$, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide, $R_4$ désigne un radical alkyle, linéaire ou ramifié, comportant de 16 à 30 atomes de carbone.
De préférence le tensioactif cationique est un sel (par exemple chlorure) de béhényl triméthyl ammonium.

ii) les radicaux $R_1$ et $R_2$, qui peuvent être identiques ou différents, représentent un radical aliphatique, linéaire ou ramifié, comportant de 1 à 4 atomes de carbone,

ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
$R_3$ et $R_4$, identiques ou différents, désignent un radical alkyle, linéaire ou ramifié, comportant de 12 à 30 atomes de carbone, ledit radical comprenant au moins une fonction ester ou amide.
$R_3$ et $R_4$ sont notamment choisis parmi les radicaux alkyl($C_{12}$-$C_{22}$)amido alkyle($C_2$-$C_6$), alkyl($C_{12}$-$C_{22}$)acétate ;
De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (X) suivante :

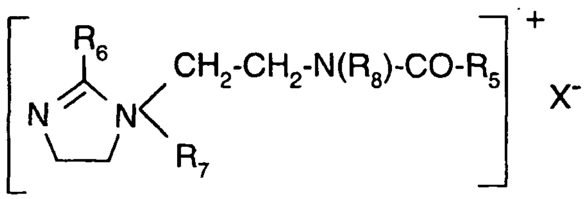

(X)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$, $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,

C) - les sels de diammonium quaternaire de formule (XI) :

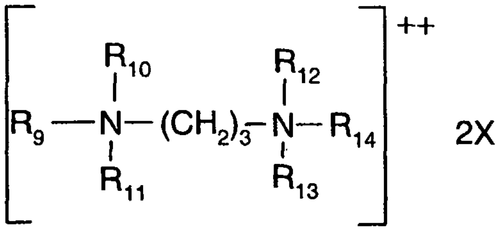 (XI)

dans laquelle $R_9$ désigne un radical aliphatique comportant environ de 16 à 30 atomes de carbone, $R_{10}$, $R_{11}$, $R_{12}$, $R_{13}$ et $R_{14}$, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (XII) suivante :

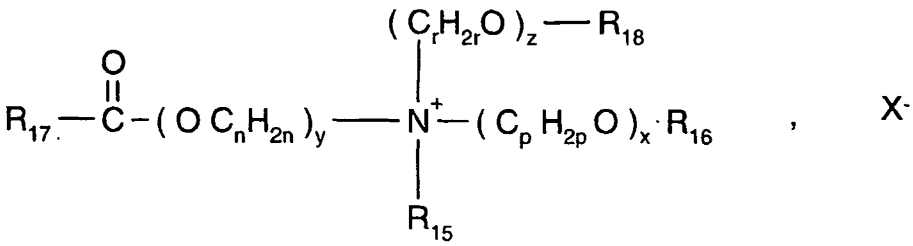 (XII)

dans laquelle :

- $R_{15}$ est choisi parmi les radicaux alkyles en $C_1$-$C_6$ et les radicaux hydroxyalkyles ou dihydroxyalkyles en $C_1$-$C_6$;
- $R_{16}$ est choisi parmi :

  - le radical

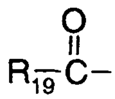

  - les radicaux $R_{20}$ hydrocarbonés en $C_1$-$C_{22}$ linéaires ou ramifiés, saturés ou insaturés,
  - l'atome d'hydrogène,

- $R_{18}$ est choisi parmi :

  - le radical

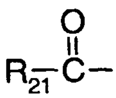

  - les radicaux $R_{22}$ hydrocarbonés en $C_1$-$C_6$ linéaires ou ramifiés, saturés ou insaturés,
  - l'atome d'hydrogène,

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;

- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

On utilise plus particulièrement les sels d'ammonium de formule (XII) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :
  - le radical

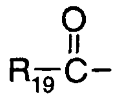

  - les radicaux méthyle, éthyle ou hydrocarbonés en $C_{14}$-$C_{22}$
  - l'atome d'hydrogène ;

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :
  - le radical

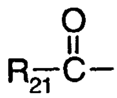

  - l'atome d'hydrogène ;

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL® 70» par la société VAN DYK, le Quaternium-27 ou le Quaternium-83 commercialisés par la société WITCO.

On utilise de préférence comme agent tensioactif anionique les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélanges avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société COGNIS ou tel que les alkyl ($C_8$-$C_{20}$) amidoalkyl ($C_1$-$C_6$) bétaïnes en particulier la TEGOBETAINE® F 50 commercialisée par la société GOLDSCHMIDT.

La composition de l'invention peut également contenir au moins un additif choisi parmi les épaississants, les agents antipelliculaires ou anti-séborrhéiques, les parfums, les agents nacrants, les hydroxyacides, les électrolytes, les conservateurs, les filtres solaires siliconés ou non, les vitamines, les provitamines tels que le panthénol, les polymères anioniques ou non ioniques, les protéines, les hydrolysats de protéines, l'acide méthyl-18 eicosanoique, ainsi que les mélanges de ces différents composés et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une basé lavante, généralement aqueuse.

Le ou les tensioactifs formant la base lavante peuvent être indifféremment choisis, seuls ou en mélanges, au sein des tensioactifs anioniques, amphotères, non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.
Lorsque la composition se présente sous la forme d'un après-shampooing éventuellement à rincer, elle contient avantageusement un tensioactif cationique, sa concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement .des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

<u>EXEMPLE 1</u>

On a réalisé un shampooing de composition suivante

| | En g MA |
|---|---|
| Lauryléthersulfate de sodium (C12/C14 - 70/30) à 2,2 moles d'oxyde d'éthylène à 70% de MA | 7 |
| Cocoylbétaine | 2.5 |
| Distéarate d'éthylèneglycol | 1.5 |

(suite)

| | En g MA |
|---|---|
| Polydiméthylsiloxane de formule (II) proposée par WACKER sous le nom SLM 28020 | 1.5 |
| Polydiméthylsiloxane de viscosité 60.000 cSt (DC200-60.000 cSt de DOW CORNING) | 1 g |
| Hydroxyéthyl cellulose quaternisée par chlorure de 2,3 epoxypropyl trimethyl ammonium vendu sous la dénomination UCARE POLYMER JR-400 par la société AMERCHOL | 0.4 |
| Polymère acrylique en émulsion vendu sous la dénomination AQUA SF1 par NOVEON | 0.8 |
| Conservateurs | q.s. |
| Agents de pH q.s. | pH 5.0 |
| Eau déminéralisée q.s.p. | 100 |

Les cheveux traités avec ce shampooing sont durablement doux et lisses.

On obtient des résultats identiques en remplaçant les 1,5 gMA de polydiméthylsiloxane de formule (II) par 1 gMA de polydiméthylsiloxane de formule (I) proposé par WACKER sous la dénomination BELSIL ADM 652.

EXEMPLE 2

On a préparé un après-shampooing à rincer de composition suivante :

| | |
|---|---|
| Alcool cétylique | 3,7 g |
| Alcool myristique | 0,4 g |
| Hydroxyéthylcellulose (NATROSOL 250 HHR de AQUALON) | 0,25 g |
| Mélange de myristate/palmitate/stéarate de myristyle/ cétyle/stéaryle (BLANC DE BALEINE VEGETAL de LASERSON) | 0.8 g |
| Chlorure de cétyl triméthyl ammonium en solution aqueuse à 25% de MA (DEHYQUART® A OR de COGNIS) | 2,5 g (0,62 gMA) |
| Chlorure de béhényl triméthyl ammonium en solution eau/ isopropanol à 80% de MA (GENAMIN KDM-F de CLARIANT) | 0,6 g (0,48 gMA) |
| Polydiméthylsiloxane de formule (II) proposé par WACKER sous le nom SLM 28020 | 5 g (0,85 gMA) |
| Méthosulfate (70/30 en poids) de dipalmitoyléthyl hydroxyéthyl Méthyl ammonium/ alcool cétylique /alcool stéarylique (DEHYQUART F 30 de COGNIS) | 1 g |
| Acide citrique qs | pH 3,5 ±0,5 |
| Parfum, conservateurs, qs | |
| Eau desionisée QSP | 100 g |

Les cheveux traités avec cette composition sont durablement doux et lisses.

EXEMPLE 3

On a préparé un après-shampooing à rincer de composition suivante :

| | |
|---|---|
| - Homopolymère de chlorure de méthacrylate d'éthyle triméthylammonium en émulsion inverse réticulé (SALCARE SC96 de CIBA) | 0.5 |
| - Phosphate de diamidon de maïs hydroxypropyle | 3 |
| - Huile de ricin hydrogénée oxyéthylénée (40 OE) | 0.5 |
| - Polydiméthylsiloxane de formule (II) proposée par WACKER sous le nom SLM 28020 | 2 |
| - Parfum | qs |
| - Conservateurs | qs |
| - Eau déminéralisée | qsp 100 g |

Les cheveux traités avec cet après-shampooing sont durablement doux et lisses.

EXEMPLE 4

On a préparé un après-shampooing à rincer de composition suivante :

| | En g MA |
|---|---|
| - Polymère SMDI / polyéthylèneglycol à terminaison décyle (ACULYN 44 de ROHM&HAAS) | 1 |
| - Homopolymère de chlorure de méthacrylate d'éthyle triméthylammonium en émulsion inverse réticulé - (SALCARE SC 96 de CIBA) | 0.2 |
| - Huile de ricin hydrogénée oxyéthylénée (40 OE) | 0.5 |
| - Polydiméthylsiloxane de formule (II) proposée par WACKER sous le nom SLM 28020 | 2 |
| - Parfum | qs |
| - Conservateurs | qs |
| - Eau déminéralisée | qsp 100 g |

Les cheveux traités avec cet après-shampooing sont durablement doux et lisses.

EXEMPLE 5

On a préparé une mousse de soin non rincée présentée en aérosol à partir de 95 g de la composition de l'exemple 3 et de 5 g de propulseur isobutane / propane / butane (56/24/20) PROPEL 45 de la société REPSOL.
Les cheveux traités avec cette mousse sont durablement doux et lisses.

## Revendications

1. Composition cosmétique, **caractérisée par le fait qu'**elle comprend, dans un milieu cosmétiquement acceptable, au moins un agent conditionneur et au moins une silicone aminée choisie parmi celles de formules (I) ou (II) suivantes :

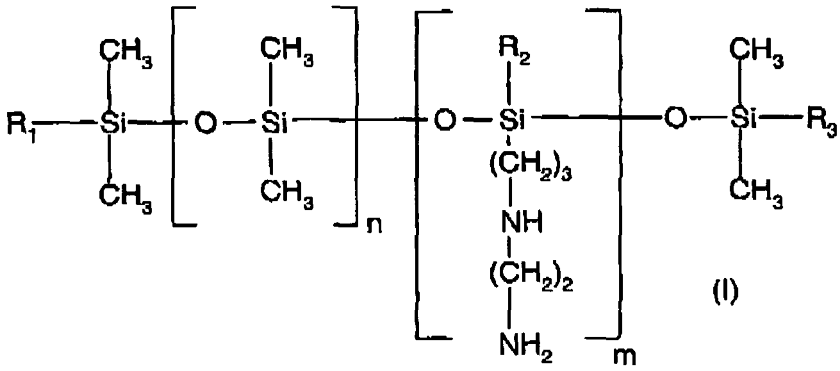

(I)

dans laquelle:

m et n sont des nombres tels que la somme (n + m) peut varier de 1 à 1 000,
n pouvant désigner un nombre de 0 à 999 et m pouvant désigner un nombre de 1 à 1 000,
$R_1$ $R_2$, $R_3$, identiques ou différents, représentent un radical hydroxy ou alcoxy en $C_1$-$C_4$, l'un au moins des radicaux $R_1$ à $R_3$ désignant un radical alcoxy.

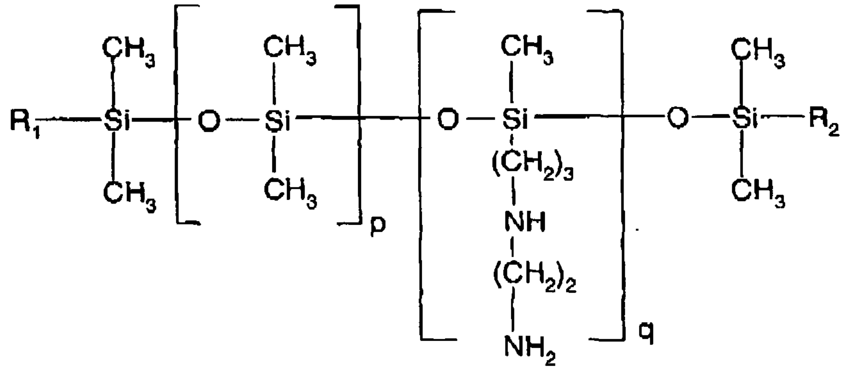

(II).

dans laquelle
p et q sont des nombres tels que la somme (p + q) peut varier de 1 à 1 000,
p pouvant désigner un nombre de 0 à 999 et q pouvant désigner un nombre de 1 à 1 000 ;
$R_1$, $R_2$, différents, représentent un radical hydroxy ou alcoxy en $C_1C_4$, l'un au moins des radicaux $R_1$ ou $R_2$ désignant un radical alcoxy.

2. Composition selon la revendication 1, **caractérisée par le fait que** le radical alcoxy en $C_1$-$C_4$ est un radical méthoxy.

3. Composition selon l'une quelconque des revendications 1 ou 2, **caractérisée par le fait que** dans les silicones aminées de formule (I le rapport molaire Hydroxy/Alcoxy va de 0,2:1 à 0,4:1.

4. Composition selon l'une quelconque des revendications 1 à 3, **caractérisée par le fait que** la masse moléculaire moyenne en poids de la silicone de formule (I) va de 2,000 à 1,000.000.

5. Composition selon l'une quelconque des revendications 1 ou 2, **caractérisée par le fait que** dans les silicones aminées de formule (II) le rapport molaire Hydroxy/Alcoxy va de 1:0,8 à 1:1,1.

6. Composition selon l'une quelconque des revendications 1,2 ou 5, **caractérisée par le fait que** la masse moléculaire moyenne en poids de la silicone de formule (II) va de 2.000 à 200.000.

7. Composition selon l'une quelconque des revendications 1 à 6, **caractérisée par le fait que** la silicone aminée est sous forme d'émulsion huile dans eau.

8. Composition selon la revendication 7, **caractérisée par le fait que** l'émulsion huile dans eau comprend un ou plusieurs tensioactifs cationiques et/ou non ioniques.

9. Composition selon les revendications 7 ou 8, **caractérisée par le fait que** la taille moyenne en nombre des particules de silicone dans l'émulsion va de 3 nm à 500 nanomètres.

10. Composition selon la revendications 9, **caractérisée par le fait que** la taille moyenne des particules de silicone dans l'émulsion va de 5 nm à 60 nanomètres .

11. Composition selon l'une quelconque des revendications 1 à 10, **caractérisée par le fait que** la ou les silicones aminées de formule (I) ou (II) sont présentes à une concentration allant de 0,01 à 20% en poids du poids total de la composition.

12. Composition selon la revendication 11, **caractérisée par le fait que** la ou les silicones aminées de formule (I) ou (II) sont présentes à une concentration allant de 0,1 % à 15 % en poids par rapport au poids total de la composition .

13. Composition selon l'une quelconque des revendications précédentes, **caractérisée en ce que** les agents conditionneurs sont choisis parmi les huiles de synthèses, les huiles minérales, les huiles végétales, les huiles fluorées ou perfluorées, les cires naturelles ou synthétiques, les composés de type céramide, les esters d'acides carboxyliques, les silicones différentes de celles de formules (I) ou (II), les polymères anioniques, les polymères non ioniques,

les polymères cationiques, les polymères amphotères, les protéines cationiques, les hydrolysats de protéines cationiques et les tensioactifs cationiques ainsi que les mélanges de ces différents composés.

**14.** Composition selon la revendication 13, **caractérisée par le fait que** la ou les huiles de synthèse sont des polyoléfines de type polybutène, hydrogéné ou non, ou de type polydécène, hydrogéné ou non.

**15.** Composition selon la revendication 13, **caractérisée par le fait que** la ou les huiles végétales sont choisies dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huilés végétales ou animales de formule $R_9COOR_{10}$ dans laquelle $R_9$ représente le reste d'un acide gras supérieur comportant de 7 à 29 atomes de carbone et $R_{10}$ représente une chaîne hydrocarbonée linéaire ou ramifiée contenant de 3 à 30 atomes de carbone, naturelles ou synthétiques de

**16.** Composition selon la revendication 13, **caractérisée par le fait que** la cire ou les cires sont choisies parmi la cire de Carnauba, la cire de Candelila, la cire d'Alfa, la cire de paraffine, l'ozokérite, les cires végétales que , cires animales, les cires de polyéthylène ou de polyoléfines.

**17.** Composition selon la revendication 14, **caractérisée par le fait que** les composés de type céramides sont choisis parmi :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol ,
- le 2-N-palmitoytamino-hexadécane-7,3-diol,
- le (bis-(N-hydroxyéthyl N-cétyl) malonamide),
- le N-(2-hydroxyéthyl)-N-(3-cétyloxy-2-hydroxyprapyl)amide d'acide cétylique,
- le N-docosanoyl N-méthyl-D-glucamine

ou les mélanges de ces composés.

**18.** Composition selon la revendication 13, **caractérisée en ce que** les esters d'addes carboxyliques sont choisis parmi les palmitates d'éthyle et d'isopropyle, le palmitate d'éthyl-2-héxyle, le palmitate de 2-octyldécyle, les myristates d'alkyles , le stéarate d'hexyle, le stéarate de butyle, le stéarate d'isobutyle; le malate de dioctyle, le laurate d'hexyle, le laurate de 2-hexyldécyle et l'isononanate d'isononyle, l'octanoate de cétyle.

**19.** Composition selon la revendication 13, **caractérisée par le fait que** les silicones différentes de celles de formules (I) ou (II) sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

**20.** Composition selon la revendication 19, **caractérisée par le fait que** :

(a) les polyalkylsiloxanes sont choisis parmi :

- les polydiméthylsiloxanes à groupements terminaux triméthylsilyle ;
- les polydiméthylsiloxanes à groupements terminaux diméthylsilanol ;
- les polyalkyl($C_1$-$C_{20}$)siloxanes ;

(b) les polyalkylarylsiloxanes sont choisis parmi :

- les polydiméthylméthylphényisiloxanes, les polydiméthyldiphényl siloxanes linéaires et/ou ramifiés de viscosité comprise entre $1.10^{-5}$ et $5.10^{-2}m^2/s$ à 25°C ;

(c) les gommes de silicone sont choisies parmi les polydiorganosiloxanes ayant des masses moléculaires moyennes en nombre comprises entre 200 000 et 1 000 000 utilisés seuls ou sous forme de mélange dans un

solvant ;

(d) les résines sont choisies parmi les résines constituées d'unités : $R_3$ Si $O_{1/2}$, $R_2$ Si $O_{2/2}$, R Si $O_{3/2}$, Si $O_{4/2}$ dans lesquelles R représente un groupement hydrocarboné ayant de 1 à 16 atomes de carbone ou un groupement phényle ;

(e) les silicones organo modifiées sont choisies parmi les silicones comportant dans leur structure un ou plusieurs groupements organofonctionnels fixés par l'intermédiaire d'un radical hydrocarboné.

**21.** Composition selon la revendication 20, **caractérisée par le fait que** Les gommes de silicone utilisées seules ou sous forme de mélange sont choisies parmi les structures suivantes :

- polydiméthylsiloxane
- polydiméthylsiloxane/méthylvinylsiloxanes,
- polydiméthylsiloxane/diphénylsiloxane,
- polydiméthylsiloxane/phénylméthylsiloxane,
- polydiméthylsiloxane/diphénylsiloxane/méthylvinylsiloxanes et les mélanges suivants :
- des mélanges formés à partir d'un polydiméthylsiloxane hydroxylé en bout de chaîne et d'un polydiméthylsiloxane cyclique ;
- les mélanges formés à partir d'une gomme polydiméthylsiloxane et d'une silicone cyclique ; et
- des mélanges de polydiméthylsiloxanes de viscosités différentes.

**22.** Composition selon la revendication 20, **caractérisée par le fait que** les silicones organomodifiées sont choisies parmi les polyorganosiloxanes comportant :

a) des groupements polyéthylèneoxy et/ou polypropylèneoxy ;
b) des groupements aminés substitués ou non ;
c) des groupements thiols ;
d) des groupements alcoxylés,
e) des groupements hydroxyalkyle,
f) des groupements acyloxyalkyla,
g) des groupements alkyl carboxyliques,
h) des groupements 2-hydroxyalkylsulfonates,
i) des groupements 2-hydroxyalkylthiosulfonates,
j) des groupements hydroxyacylamino.

**23.** Composition selon l'une quelconque des revendication 20 à 22, **caractérisée par le fait que** les silicones sont choisis parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

**24.** Composition selon la revendication 13, **caractérisée par le fait que** le polymère anionique est choisi parmi :

- les polymères comportant des motifs carboxyliques dérivant de monomères mono ou diacides carboxyliques insaturés de formule :

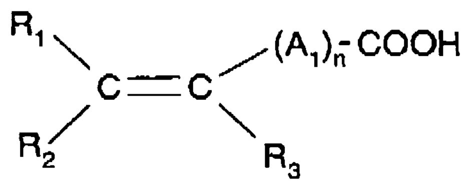

(III)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre. $R_1$ désigne un atome d'hydrogène, un groupement phényle ou benzyle, $R_2$ désigne un atome d'hydrogène, un groupement alkyle inférieur ou carboxyle,

$R_3$ désigne un atome d'hydrogène, un groupement alkyle Inférieur, un groupement $-CH_2-COOH$, phényle ou benzyle ;
- les polymères comprenant des motifs dérivant d'acide sulfonique tels que des motifs vinylsulfonique, styrènesulfonique, acrylamido alkylsulfonique.

**25.** Composition selon la revendication précédente, **caractérisée en ce que** le polymère anionique est choisi parmi :

- les copolymères d'acide acrylique ;
- les copolymères dérivés d'acide crotonique ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ;
- les copolymères d'acide méthacrylique et de méthacrylate de méthyle ;
- le copolymère d'acide méthacrylique et d'acrylate d'éthyle;
- le copolymère acétate de vinyle/acide crotonique ;
- le terpolymère acétate de vinyle/acide crotonique/polyéthylèneglycol.

**26.** Composition selon la revendication 13, **caractérisée en ce que** le polymère amphotère est choisi parmi les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylamino-éthyle avec le sulfate de diméthyle ou diéthyle.

**27.** Composition selon la revendication 13, **caractérisée par le fait que** le polymère non ionique est choisi parmi :

- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques
- les copolymères d'acrylonitrile et d'un monomère non ionique ; et
- les copolymères d'acrylate d'alkyle et d'uréthanne.

**28.** Composition selon la revendication 13, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être : portés par un substituant latéral directement relié à celle-ci.

**29.** Composition selon la revendication 28, **caractérisée par le fait que** ledit polymère cationique est choisi parmi les cyclopolymères cationiques, les polysaccharides cationiques, les polymères quaternaires de vinylpyrrolidone et de vinylimidazole et leurs mélanges.

**30.** Composition selon la revendication 29, **caractérisée par le fait que** ledit cyclopolymère est choisi parmi les homopolymères du chlorure de diallyldiméthylammonium et les copolymères du chlorure de diallyldiméthylammonium et d'acrylamide.

**31.** Composition selon la revendication 29, **caractérisée par le fait que** lesdites polysaccharides cationiques sont choisis parmi les hydroxyéthylcelluloses ayant réagi avec un époxyde substitué par un groupement triméthylammonium.

**32.** Composition selon la revendication 29, **caractérisée par le fait que** lesdits polysaccharides cationiques sont choisis parmi les gommes de guar modifiées par un sel de 2,3-époxypropyl triméthylammonium.

**33.** Composition selon la revendication 13, **caractérisée par le fait que** les tensioactifs cationiques sont choisis parmi :

A) - les sels d'ammonium quaternaires de la formule générale (XXIII) suivante :

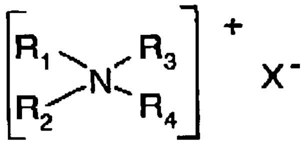

(XXIII)

dans laquelle X est un anion choisi dans le groupe des halogénures, ou alkyl($C_2$-$C_6$)sulfates des phosphates, des alkyl-ou-alkylarylsulfonates, des anions dérivés d'acide organique
, et

i) les radicaux R1 à R3, qui peuvent être identiques ou différents, représentent un radical aliphatique, linéaire ou ramifié, comportant de 1 à 4 atomes de carbone, ou un radical aromatique, les radicaux aliphatiques pouvant comporter des hétéroatomes , R4 désigne un radical alkyle, linéaire ou ramifié, comportant de 16 à 30 atomes de carbone.
ou
ii) les radicaux R1 et R2, qui peuvent être identiques ou différents, représentent un radical aliphatique, linéaire ou ramifié, comportant de 1 à 4 atomes de carbone, ou un radical aromatique les radicaux aliphatiques pouvant comporter des hétéroatomes R3 et R4, identiques ou différents, désignent un radical alkyle, linéaire ou ramifié, comportant de 12 à 30 atomes de carbone, ledit radical comprenant au moins une fonction ester ou amide.

B) - les sels d'ammonium quaternaire de l'imidazolinium,
C) - les sels de diammonium quaternaire de formule (XXV) :

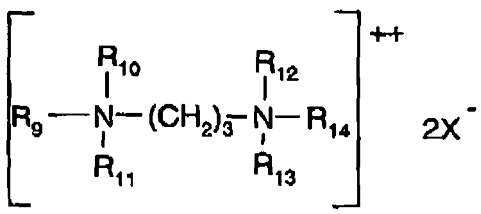

(XXV)

dans laquelle $R_9$ désigne un radical aliphatique comportant environ de 16 à 30 atomes de carbone, $R_{10}$, $R_{11}$, $R_{12}$, $R_{13}$ et $R_{14}$, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates.
D) - les sels d'ammonium quaternaire contenant au moins une fonction ester de formule (XXVI) suivante :

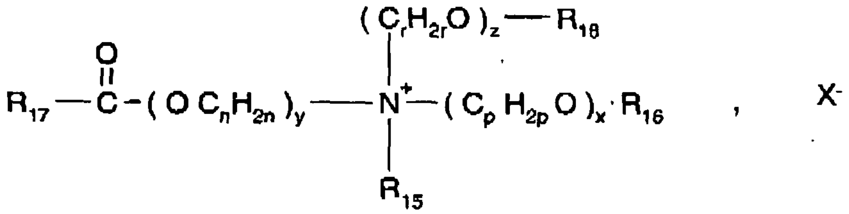

(XXVI)

dans laquelle :

- $R_{15}$ est choisi parmi les radicaux alkyles en $C_1$-$C_6$ et les radicaux hydroxyalkyles ou dihydroxyalkyles en

$C_1$-$C_6$ ;
- $R_{16}$ est choisi parmi :
- le radical

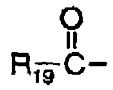

- les radicaux $R_{20}$ hydrocarbonés en $C_1$-$C_{22}$ linéaires ou ramifiés, saturés ou insaturés,
- l'atome d'hydrogène,
- $R_{18}$ est choisi parmi :
- le radical

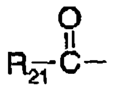

- les radicaux $R_{22}$ hydrocarbonés en $C_1$-$C_6$ linéaires ou ramifiés, saturés ou insaturés,
- l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entrer valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15, que lorsque x vaut 0 alors $R_{16}$ désigne $R_{20}$ et que lorsque z vaut 0 alors $R_{18}$ désigne $R_{22}$.

**34.** Composition selon l'une quelconque des revendications précédentes, **caractérisée en ce que** l'agent conditionneur est présent à une concentration allant de 0,001 % à 20 % en poids par rapport au poids total de la composition .

**35.** Composition selon l'une quelconque des revendications précédentes, **caractérisée en ce qu'**elle comprend au moins un polymère cationique et/ou au moins une silicone,

**36.** Composition selon l'une quelconque des revendications précédentes, **caractérisée par le fait qu'**elle comprend en outre au moins un agent tensioactif choisi parmi les tensioactifs anioniques, non ioniques, amphotères, cationiques et leurs mélanges.

**37.** Composition selon la revendication 36, **caractérisée par le fait que** le ou les agents tensioactifs sont présents à une concentration comprise entre 0,1% et 60% en poids , par rapport au poids total de la composition.

**38.** Composition selon l'une quelconque des revendications 1 à 37, **caractérisée par** la fait la composition contenir au moins un additif choisi parmi les épaississants, les agents antipelliculaires ou anti-séborrhéiques, les parfums, les agents nacrants, les hydroxyacides, les électrolytes, les conservateurs, les filtres solaires siliconés ou non, les vitamines, les provitamines, les polymères anioniques ou non ioniques, les protéines, les hydrolysats de protéines, l'acide méthyl-18 eicosanoique, le panthénol, ainsi que les mélanges de ces différents composés.

**39.** Composition selon l'une quelconque des revendications précédentes, **caractérisée par** la fait qu'elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

**40.** Utilisation d'une composition telle que définie dans l'une quelconque des revendications précédentes pour le lavage ou pour le soin des matières kératiniques.

**41.** Procédé de traitement des matières kératiniques, telles que les cheveux, **caractérisé en ce qu'**il consiste à appliquer sur lesdites matières une composition cosmétique selon l'une des revendications 1 à 39, puis à effectuer éventuellement un rinçage à l'eau.

**42.** Utilisation d'une composition selon l'une des revendications 1 à 39 pour le conditionnement des matières kératiniques.

**43.** Utilisation d'une composition selon l'une des revendications 1 à 39 pour améliorer la légèreté, la douceur, la brillance et/ou le démêlage, pour faciliter le coiffage des matières kératiniques.

**44.** Utilisation d'une composition selon l'une des revendications 1 à 39 pour améliorer la rémanence des effets du conditionnement aux shampooings.

**45.** Utilisation d'au moins une silicone aminée telle que définie selon l'une des revendications 1 à 12 pour améliorer la rémanence des effets du conditionnement d'une composition cosmétique aux shampooings.

**46.** Procédé pour améliorer la rémanence des effets du conditionnement d'une composition cosmétique aux shampooings qui consiste à introduire la silicone aminée telle que définie selon l'une des revendications 1 à 12 dans une composition cosmétique.

## Claims

**1.** Cosmetic composition, **characterized in that** it comprises, in a cosmetically acceptable medium, at least one conditioner and at least one aminosilicone chosen from those of formula (I) or (II) below:

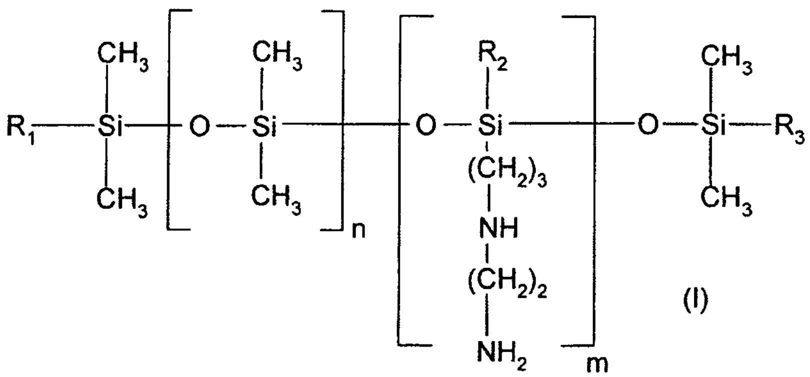

(I)

in which:

m and n are numbers such that the sum (n + m) can range from 1 to 1000,
n possibly denoting a number from 0 to 999 and m possibly denoting a number from 1 to 1000;
$R_1$, $R_2$ and $R_3$, which may be identical or different, represent a hydroxyl or $C_1$-$C_4$ alkoxy radical, at least one of the radicals $R_1$ to $R_3$ denoting an alkoxy radical;

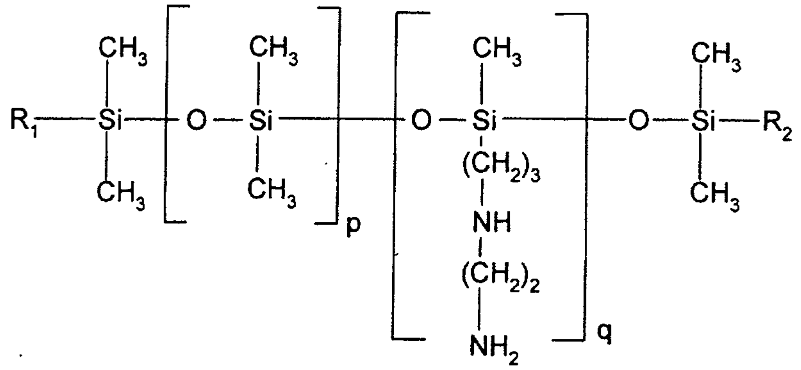

(II)

in which:

p and q are numbers such that the sum (p + q) can range from 1 to 1000,
p possibly denoting a number from 0 to 999 and q possibly denoting a number from 1 to 1000;
$R_1$ and $R_2$, which are different, represent a hydroxyl or $C_1$-$C_4$ alkoxy radical, at least one of the radicals $R_1$ and $R_2$ denoting an alkoxy radical.

2. Composition according to Claim 1, **characterized in that** the $C_1$-$C_4$ alkoxy radical is a methoxy radical.

3. Composition according to either of Claims 1 and 2, **characterized in that**, in the aminosilicones of formula (I), the hydroxyl/alkoxy molar ratio ranges from 0.2:1 to 0.4:1.

4. Composition according to any one of Claims 1 to 3, **characterized in that** the silicone of formula (I) has a weight-average molecular mass ranging from 2000 to 1 000 000.

5. Composition according to either of Claims 1 and 2, **characterized in that**, in the aminosilicones of formula (II), the hydroxyl/alkoxy molar ratio ranges from 1:0.8 to 1:1.1.

6. Composition according to any one of Claims 1, 2 and 5, **characterized in that** the silicone of formula (II) has a weight-average molecular mass ranging from 2000 to 200 000.

7. Composition according to any one of Claims 1 to 6, **characterized in that** the aminosilicone is in the form of an oil-in-water emulsion.

8. Composition according to Claim 7, **characterized in that** the oil-in-water emulsion comprises one or more cationic and/or nonionic surfactants.

9. Composition according to Claim 7 or 8, **characterized in that** the number-average size of the silicone particles in the emulsion ranges from 3 nm to 500 nanometres.

10. Composition according to Claim 9, **characterized in that** the average size of the silicone particles in the emulsion ranges from 5 nm to 60 nanometres.

11. Composition according to any one of Claims 1 to 10, **characterized in that** the aminosilicone(s) of formula (I) or (II) is (are) present at a concentration ranging from 0.01% to 20% by weight relative to the total weight of the composition.

12. Composition according to Claim 11, **characterized in that** the aminosilicone(s) of formula (I) or (II) is (are) present at a concentration ranging from 0.1% to 15% by weight relative to the total weight of the composition.

13. Composition according to any one of the preceding claims, **characterized in that** the conditioners are chosen from synthetic oils, mineral oils, plant oils, fluoro oils or perfluoro oils, natural or synthetic waxes, compounds of ceramide type, carboxylic acid esters, silicones other than those of formula (I) or (II), anionic polymers, nonionic polymers,

cationic polymers, amphoteric polymers, cationic proteins, cationic protein hydrolysates and cationic surfactants, and also mixtures of these various compounds.

**14.** Composition according to Claim 13, **characterized in that** the synthetic oil(s) is (are) polyolefins of hydrogenated or non-hydrogenated polybutene type or of hydrogenated or non-hydrogenated polydecene type.

**15.** Composition according to Claim 13, **characterized in that** the plant oil(s) is (are) chosen from the group formed by sunflower oil, corn oil, soybean oil, avocado oil, jojoba oil, marrow oil, grapeseed oil, sesame oil, hazelnut oil, fish oils, glyceryl tricaprocaprylate, or plant or animal oils of formula $R_9COOR_{10}$ in which $R_9$ represents a higher fatty acid residue containing from 7 to 29 carbon atoms and $R_{10}$ represents a linear or branched hydrocarbon-based chain containing from 3 to 30 carbon atoms and natural or synthetic essential oils.

**16.** Composition according to Claim 13, **characterized in that** the wax(es) is (are) chosen from carnauba wax, candelilla wax, alfalfa wax, paraffin wax, ozokerite, plant waxes, animal waxes, polyethylene waxes and polyolefins.

**17.** Composition according to Claim 13, **characterized in that** the compounds of ceramide type are chosen from:

- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol,
- 2-N-palmitoylaminohexadecane-1,3-diol,
- bis(N-hydroxyethyl-N-cetyl)malonamide,
- N-(2-hydroxyethyl)-N-(3-cetyloxy-2-hydroxypropyl)cetylamide,
- N-docosanoyl-N-methyl-D-glucamine or mixtures of these compounds.

**18.** Composition according to Claim 13, **characterized in that** the carboxylic acid esters are chosen from ethyl and isopropyl palmitates, 2-ethylhexyl palmitate, 2-octyldecyl palmitate, alkyl myristates, hexyl stearate, butyl stearate, isobutyl stearate; dioctyl malate, hexyl laurate, 2-hexyldecyl laurate, isononyl isononanoate and cetyl octanoate.

**19.** Composition according to Claim 13, **characterized in that** the silicones other than those of formula (I) or (II) are non-volatile polyorganosiloxanes chosen from polyalkylsiloxanes, polyarylsiloxanes, polyalkylarylsiloxanes, silicone gums and resins, polyorganosiloxanes modified with organofunctional groups, and mixtures thereof.

**20.** Composition according to Claim 19, **characterized in that**:

(a) the polyalkylsiloxanes are chosen from:

- polydimethylsiloxanes containing trimethylsilyl end groups;
- polydimethylsiloxanes containing dimethylsilanol end groups;
- poly($C_1$-$C_{20}$)alkylsiloxanes;

(b) the polyalkylarylsiloxanes are chosen from:

- linear and/or branched polydimethylmethylphenyl-siloxanes and polydimethyldiphenylsiloxanes with a viscosity of between $1\times10^{-5}$ and $5\times10^{-2}$ $m^2/s$ at 25°C;

(c) the silicone gums are chosen from polydiorganosiloxanes with number-average molecular masses of between 200 000 and 1 000 000, used alone or in the form of a mixture in a solvent;
(d) the resins are chosen from resins consisting of the following units: $R_3SiO_{1/2}$, $R_2SiO_{2/2}$, $RSiO_{3/2}$ and $SiO_{4/2}$ in which R represents a hydrocarbon-based group containing from 1 to 16 carbon atoms or a phenyl group;
(e) the organomodified silicones are chosen from silicones comprising in their structure one or more organofunctional groups attached via a hydrocarbon-based radical.

**21.** Composition according to Claim 20, **characterized in that** the silicone gums used, alone or in the form of a mixture,

are chosen from the following structures:

- polydimethylsiloxane
- polydimethylsiloxane/methylvinylsiloxane,
- polydimethylsiloxane/diphenylsiloxane,
- polydimethylsiloxane/phenylmethylsiloxane,
- polydimethylsiloxane/diphenylsiloxane/methylvinylsiloxane, and the following mixtures:
- mixtures formed from a polydimethylsiloxane hydroxylated at the end of the chain and from a cyclic polydimethylsiloxane;
- mixtures formed from a polydimethylsiloxane gum and from a cyclic silicone; and
- mixtures of polydimethylsiloxanes of different viscosities.

**22.** Composition according to Claim 20, **characterized in that** the organomodified silicones are chosen from polyorganosiloxanes comprising:

a) polyethyleneoxy and/or polypropyleneoxy groups;
b) substituted or unsubstituted amine groups;
c) thiol groups;
d) alkoxylated groups;
e) hydroxyalkyl groups;
f) acyloxyalkyl groups;
g) alkylcarboxylic groups;
h) 2-hydroxyalkylsulfonate groups;
i) 2-hydroxyalkylthiosulfonate groups;
j) hydroxyacylamino groups.

**23.** Composition according to any one of Claims 20 to 22, **characterized in that** the silicones are chosen from polyalkylsiloxanes containing trimethylsilyl end groups, polyalkylsiloxanes containing dimethylsilanol end groups, polyalkylarylsiloxanes, mixtures of two PDMSs consisting of a gum and an oil of different viscosities, mixtures of organosiloxanes and of cyclic silicones, and polyorganosiloxane resins.

**24.** Composition according to Claim 13, **characterized in that** the anionic polymer is chosen from:

- polymers comprising carboxylic units derived from unsaturated mono- or dicarboxylic acid monomers of formula:

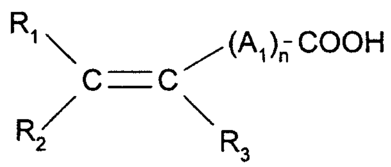

(III)

in which n is an integer from 0 to 10, $A_1$ denotes a methylene group, optionally connected to the carbon atom of the unsaturated group, or to the neighbouring methylene group when n is greater than 1, via a heteroatom such as oxygen or sulfur, $R_1$ denotes a hydrogen atom or a phenyl or benzyl group, $R_2$ denotes a hydrogen atom or a lower alkyl or carboxyl group, $R_3$ denotes a hydrogen atom, a lower alkyl group or a
- $CH_2$-COOH, phenyl or benzyl group;
- polymers comprising units derived from sulfonic acid, such as vinylsulfonic, styrenesulfonic and acrylamidoalkylsulfonic units.

**25.** Composition according to the preceding claim, **characterized in that** the anionic polymer is chosen from:

- acrylic acid copolymers;
- copolymers derived from crotonic acid;
- polymers derived from maleic, fumaric or itaconic acids or anhydrides with vinyl esters, vinyl ethers, vinyl halides or phenylvinyl derivatives, acrylic acid and its esters;
- copolymers of methacrylic acid and of methyl methacrylate;

- the copolymer of methacrylic acid and of ethyl acrylate;
- vinyl acetate/crotonic acid copolymer;
- vinyl acetate/crotonic acid/polyethylene glycol terpolymer.

**26.** Composition according to Claim 13, **characterized in that** the amphoteric polymer is chosen from polymers comprising units derived from:

a) at least one monomer chosen from acrylamides and methacrylamides substituted on the nitrogen with an alkyl radical,
b) at least one acidic comonomer containing one or more reactive carboxylic groups, and from
c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate.

**27.** Composition according to Claim 13, **characterized in that** the nonionic polymer is chosen from:

- polyalkyloxazolines;
- vinyl acetate homopolymers;
- copolymers of vinyl acetate and acrylic ester;
- copolymers of vinyl acetate and ethylene;
- copolymers of vinyl acetate and maleic ester;
- copolymers of polyethylene and maleic anhydride;
- alkyl acrylate homopolymers and alkyl methacrylate homopolymers;
- acrylic ester copolymers;
- copolymers of acrylonitrile and a nonionic monomer; and
- copolymers of alkyl acrylate and of urethane.

**28.** Composition according to Claim 13, **characterized in that** the cationic polymers are chosen from those containing units comprising primary, secondary, tertiary and/or quaternary amine groups that can either form part of the main polymer chain or be borne by a side substituent directly attached to the said chain.

**29.** Composition according to Claim 28, **characterized in that** the said cationic polymer is chosen from cationic cyclopolymers, cationic polysaccharides, quaternary polymers of vinylpyrrolidone and of vinylimidazole, and mixtures thereof.

**30.** Composition according to Claim 29, **characterized in that** the said cyclopolymer is chosen from diallyldimethylammonium chloride homopolymers and copolymers of diallyldimethylammonium chloride and of acrylamide.

**31.** Composition according to Claim 29, **characterized in that** the said cationic polysaccharides are chosen from hydroxyethylcelluloses that have reacted with an epoxide substituted with a trimethylammonium group.

**32.** Composition according to Claim 29, **characterized in that** the said cationic polysaccharides are chosen from guar gums modified with a 2,3-epoxypropyltrimethylammonium salt.

**33.** Composition according to Claim 13, **characterized in that** the cationic surfactants are chosen from:

A) - the quaternary ammonium salts of general formula (XXIII) below:

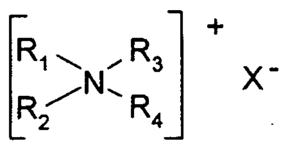

(XXIII)

in which $X^-$ is an anion chosen from the group of halides or ($C_2$-$C_6$)alkyl sulfates, phosphates, alkyl or alkylaryl sulfonates, anions derived from organic acid, and

i) the radicals $R_1$ to $R_3$, which may be identical or different, represent a linear or branched aliphatic radical containing from 1 to 4 carbon atoms, or an aromatic radical, it being possible for the aliphatic radicals to comprise heteroatoms,

$R_4$ denotes a linear or branched alkyl radical containing from 16 to 30 carbon atoms, or

ii) the radicals $R_1$ and $R_2$, which may be identical or different, represent a linear or branched aliphatic radical containing from 1 to 4 carbon atoms, or an aromatic radical, it being possible for the aliphatic radicals to comprise heteroatoms,

$R_3$ and $R_4$, which may be identical or different, denote a linear or branched alkyl radical containing from 12 to 30 carbon atoms, the said radical comprising at least one ester or amide function;

B) - the quaternary ammonium salts of imidazolinium;

C) - the diquaternary ammonium salts of formula (XXV) :

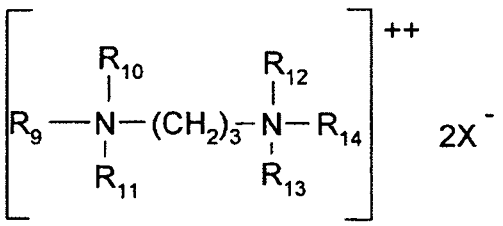

(XXV)

in which $R_9$ denotes an aliphatic radical containing from about 16 to 30 carbon atoms, $R_{10}$, $R_{11}$, $R_{12}$, $R_{13}$ and $R_{14}$, which may be identical or different, are chosen from hydrogen and an alkyl radical containing from 1 to 4 carbon atoms, and $X^-$ is an anion chosen from the group of halides, acetates, phosphates, nitrates and methyl sulfates;

D) - the quaternary ammonium salts containing at least one ester function, of formula (XXVI) below:

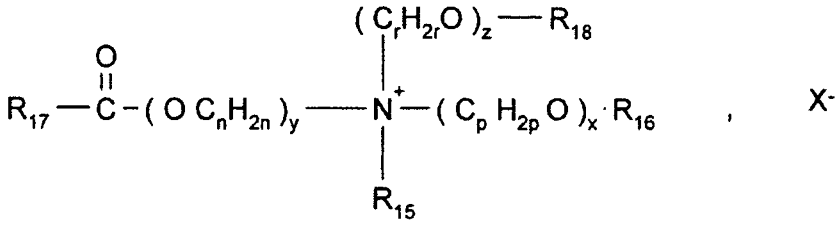

(XXVI)

in which:

- $R_{15}$ is chosen from $C_1$-$C_6$ alkyl radicals and $C_1$-$C_6$ hydroxyalkyl or dihydroxyalkyl radicals;
- $R_{16}$ is chosen from:
- a radical

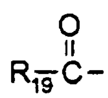

- linear or branched, saturated or unsaturated $C_1$-$C_{22}$ hydrocarbon-based radicals $R_{20}$,
- a hydrogen atom,
- $R_{18}$ is chosen from:
- a radical

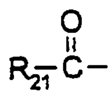

- linear or branched, saturated or unsaturated $C_1$-$C_6$ hydrocarbon-based radicals $R_{22}$,
- a hydrogen atom,
- $R_{17}$, $R_{19}$ and $R_{21}$, which may be identical or different, are chosen from linear or branched, saturated or unsaturated $C_7$-$C_{21}$ hydrocarbon-based radicals;
- n, p and r, which may be identical or different, are integers ranging from 2 to 6;
- y is an integer ranging from 1 to 10;
- x and z, which may be identical or different, are integers ranging from 0 to 10;
- $X^-$ is a simple or complex, organic or inorganic anion;

with the proviso that the sum x + y + z is from 1 to 15, that when x is 0, then $R_{16}$ denotes $R_{20}$ and that when z is 0, then $R_{18}$ denotes $R_{22}$.

**34.** Composition according to any one of the preceding claims, **characterized in that** the conditioner is present at a concentration ranging from 0.001% to 20% by weight relative to the total weight of the composition.

**35.** Composition according to any one of the preceding claims, **characterized in that** it comprises at least one cationic polymer and/or at least one silicone.

**36.** Composition according to any one of the preceding claims, **characterized in that** it also comprises at least one surfactant chosen from anionic, nonionic, amphoteric and cationic surfactants, and mixtures thereof.

**37.** Composition according to Claim 36, **characterized in that** the surfactant(s) is (are) present at a concentration of between 0.1% and 60% by weight relative to the total weight of the composition.

**38.** Composition according to any one of Claims 1 to 37, **characterized in that** the composition contains at least one additive chosen from thickeners, antidandruff or anti-seborrhoeic agents, fragrances, nacreous agents, hydroxy acids, electrolytes, preserving agents, silicone or non-silicone sunscreens, vitamins, provitamins, anionic or nonionic polymers, proteins, protein hydrolysates, 18-methyleicosanoic acid and panthenol, and also mixtures of these various compounds.

**39.** Composition according to any one of the preceding claims, **characterized in that** it is in the form of a shampoo, a conditioner, a composition for permanent-waving, straightening, dyeing or bleaching the hair, a rinse-out composition to be applied between the two steps of a permanent-waving or hair-straightening operation, or a washing composition for the body.

**40.** Use of a composition as defined in any one of the preceding claims, for washing or caring for keratin materials.

**41.** Process for treating keratin materials, such as the hair, **characterized in that** it consists in applying to the said materials a cosmetic composition according to one of Claims 1 to 39, and then optionally in rinsing with water.

**42.** Use of a composition according to one of Claims 1 to 39, to condition keratin materials.

**43.** Use of a composition according to one of Claims 1 to 39, to improve the lightness, softness, sheen and/or disentangling, and to facilitate the styling of keratin materials.

**44.** Use of a composition according to one of Claims 1 to 39, to improve the remanence of the conditioning effects with respect to shampooing.

**45.** Use of at least one aminosilicone as defined according to one of Claims 1 to 12, for improving the remanence of the conditioning effects of a cosmetic composition with respect to shampooing.

**46.** Process for improving the remanence of the conditioning effects of a cosmetic composition with respect to shampooing, which consists in introducing the aminosilicone as defined according to one of Claims 1 to 12 into a cosmetic composition.

## Patentansprüche

1. Kosmetische Zusammensetzung, **dadurch gekennzeichnet, dass** sie in einem kosmetisch akzeptablen Medium mindestens ein Konditioniermittel und mindestens ein aminiertes Silicon enthält, das unter den Siliconen der folgenden Formeln (I) oder (II) ausgewählt ist:

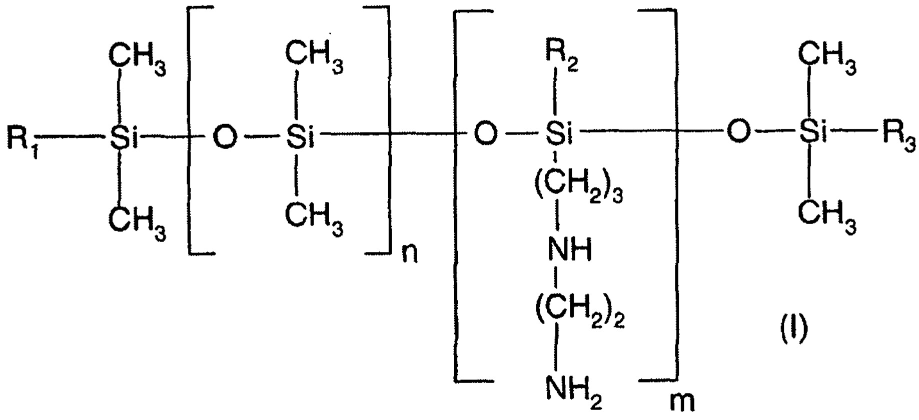

(I)

in der Formel:

m und n bedeuten Zahlen, die so gewählt sind, dass die Summe (n + m) im Bereich von 1 bis 1000 liegen kann, n kann eine Zahl im Bereich von 0 bis 999 und m eine Zahl im Bereich von 1 bis 1000 bedeuten,
$R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

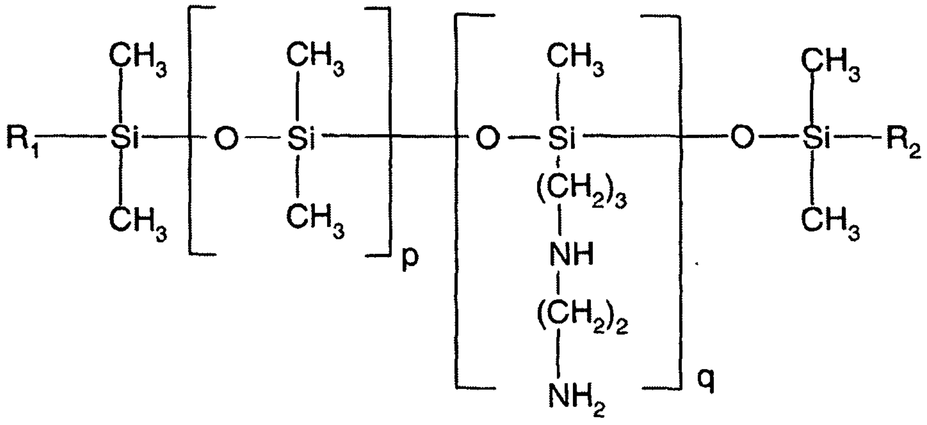

(II)

in der Formel:

p und q bedeuten Zahlen, die so gewählt sind, dass die Summe (p + q) im Bereich von 1 bis 1000 liegen kann, p kann eine Zahl im Bereich von 0 bis 999 und q eine Zahl im Bereich von 1 bis 1000 bedeuten,
$R_1$ und $R_2$, die verschieden sind, bedeuten eine Hydroxygruppe oder eine $C_{1-4}$-Alkoxygruppe, wobei mindestens eine der Gruppen $R_1$ oder $R_2$ eine Alkoxygruppe bedeutet.

2. Zusammensetzung nach Anspruch 1, **dadurch gekennzeichnet, dass** die $C_{1-4}$-Alkoxygruppe die Methoxygruppe bedeutet.

3. Zusammensetzung nach einem der Ansprüche 1 oder 2, **dadurch gekennzeichnet, dass** für die aminierten Silicone

der Formel (I) das Molverhältnis Hydroxy/Alkoxy im Bereich von 0,2:1 bis 0,4:1 liegt.

**4.** Zusammensetzung nach einem der Ansprüche 1 bis 3, **dadurch gekennzeichnet, dass** die gewichtmittlere Molmasse des Silicons der Formel (I) im Bereich von 2.000 bis 1.000.000 liegt.

**5.** Zusammensetzung nach einem der Ansprüche 1 oder 2, **dadurch gekennzeichnet, dass** für die aminierten Silicone der Formel (II) das Molverhältnis Hydroxy/Alkoxy im Bereich von 1:0,8 bis 1:1,1 liegt.

**6.** Zusammensetzung nach einem der Ansprüche 1, 2 oder 5, **dadurch gekennzeichnet, dass** die gewichtmittlere Molmasse des Silicons der Formel (II) im Bereich von 2.000 bis 200.000 liegt.

**7.** Zusammensetzung nach einem der Ansprüche 1 bis 6, **dadurch gekennzeichnet, dass** das aminierte Silicon in Form einer Öl-in-Wasser-Emulsion vorliegt.

**8.** Zusammensetzung nach Anspruch 7, **dadurch gekennzeichnet, dass** die Öl-in-Wasser-Emulsion einen oder mehrere kationische und/oder nichtionische grenzflächenaktive Stoffe enthält.

**9.** Zusammensetzung nach den Ansprüchen 7 oder 8, **dadurch gekennzeichnet, dass** die zahlenmittlere Größe der Siliconpartikel in der Emulsion im Bereich von 3 bis 500 nm liegt.

**10.** Zusammensetzung nach Anspruch 9, **dadurch gekennzeichnet, dass** die mittlere Größe der Siliconpartikel in der Emulsion im Bereich von 5 bis 60 nm liegt.

**11.** Zusammensetzung nach einem der Ansprüche 1 bis 10, **dadurch gekennzeichnet, dass** das oder die aminierte (n) Silicon(e) der Formel (I) oder (II) in einer Konzentration von 0,01 bis 20 Gew.-% des Gesamtgewichts der Zusammensetzung enthalten sind.

**12.** Zusammensetzung nach Anspruch 11, **dadurch gekennzeichnet, dass** das oder die aminierte(n) Silicon(e) der Formel (I) oder (II) in einer Konzentration im Bereich von 0,1 bis 15 Gew.-% des Gesamtgewichts der Zusammensetzung enthalten sind.

**13.** Zusammensetzung nach einem der vorhergehenden Ansprüche, **dadurch gekennzeichnet, dass** die Konditioniermittel unter den synthetischen Ölen, Mineralölen, pflanzlichen Ölen, fluorierten oder perfluorierten Ölen, natürlichen oder synthetischen Wachsen, Verbindungen vom Ceramidtyp, Carbonsäureestern, von den Siliconen der Formeln (I) oder (II) verschiedenen Siliconen, anionischen Polymeren, nichtionischen Polymeren, kationischen Polymeren, amphoteren Polymeren, kationischen Proteinen, kationischen Proteinhydrolysaten und kationischen grenzflächenaktiven Stoffen sowie den Gemischen dieser verschiedenen Verbindungen ausgewählt sind.

**14.** Zusammensetzung nach Anspruch 13, **dadurch gekennzeichnet, dass** das oder die synthetischen Öle Polyolefine vom hydrierten oder nicht hydrierten Polybutentyp oder vom hydrierten oder nicht hydrierten Polydecentyp sind.

**15.** Zusammensetzung nach Anspruch 13, **dadurch gekennzeichnet, dass** das oder die pflanzlichen Öle unter Sonnenblumenöl, Maisöl, Sojaöl, Avocadoöl, Jojobaöl, Kürbiskernöl, Traubenkernöl, Sesamöl, Haselnussöl, Fischölen, Glycerintricaprocaprylat oder pflanzlichen oder tierischen Ölen der Formel $R_9COOR_{10}$, worin $R_9$ den Rest einer höheren Fettsäure mit 7 bis 29 Kohlenstoffatomen und $R_{10}$ eine lineare oder verzweigte Kohlenwasserstoffkette mit 3 bis 30 Kohlenstoffatomen bedeutet, natürlichen oder synthetischen etherischen Ölen ausgewählt sind.

**16.** Zusammensetzung nach Anspruch 13, **dadurch gekennzeichnet, dass** das Wachs oder die Wachse unter Carnaubawachs, Candelillawachs, Alfawachs, Paraffinwachs, Ozokerit, pflanzlichen Wachsen, tierischen Waschen, Polyethylenwachsen oder Polyolefinwachsen ausgewählt sind.

**17.** Zusammensetzung nach Anspruch 13, **dadurch gekennzeichnet, dass** die Verbindungen von Ceramidtyp ausgewählt sind unter:

- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,

- 2-N-Behenoylamino-octadecan-1,3-diol,
- 2-N-[2-Hydroxypalmitoyl]-amino-octadecan-1,3-diol,
- 2-N-Stearoylamino-octadecan-1,3,4-triol,
- 2-N-Palmitoylamino-hexadecan-1, 3-diol,
- Bis(N-hydroxyethyl-N-cetyl)-malonamid,
- N-(2-Hydroxyethyl)-N-(3-cetyloxy-2-hydroxypropyl)amid von Cetylsäure,
- N-Docosanoyl-N-methyl-D-glucamin

oder den Gemischen dieser Verbindungen.

**18.** Zusammensetzung nach Anspruch 13, **dadurch gekennzeichnet, dass** die Carbonsäureester unter Ethylpalmitat und Isopropylpalmitat, 2-Ethylhexylpalmitat, 2-Octyldecylpalmitat, Alkylmyristaten, Hexylstearat, Butylstearat, Isobutylstearat; Dioctylmalat, Hexyllaurat, 2-Hexyldecyllaurat, Isononylisononanoat und Cetyloctanoat ausgewählt sind.

**19.** Zusammensetzung nach Anspruch 13, **dadurch gekennzeichnet, dass** die von den Siliconen der Formeln (I) oder (II) verschiedenen Silicone nichtflüchtige Polyorganosiloxane sind, die unter den Polyalkylsiloxanen, Polyarylsiloxanen, Polyalkylarylsiloxanen, Silicongummis, Siliconharzen, mit organofunktionellen Gruppen modifizierten Polyorganosiloxanen sowie deren Gemischen ausgewählt sind.

**20.** Zusammensetzung nach Anspruch 19, **dadurch gekennzeichnet, dass**:

(a) die Polyalkylsiloxane ausgewählt sind unter:

- Polydimethylsiloxanen mit endständigen Trimethylsilylgruppen;
- Polydimethylsiloxanen mit endständigen Dimethylsilanolgruppen;
- Polyalkyl($C_{1-20}$) siloxanen;

(b) die Polyarylsiloxane ausgewählt sind unter:

- Polydimethylmethylphenylsiloxanen, Polydimethyldiphenylsiloxanen, die geradkettig und/oder verzweigt vorliegen und bei 25°C eine Viskosität im Bereich von $1 \cdot 10^{-5}$ bis $5 \cdot 10^{-2}$ $m^2/s$ aufweisen;

(c) die Silicongummis unter den Polydiorganosiloxanen ausgewählt sind, die zahlenmittlere Molmassen im Bereich von 200 000 bis 1 000 000 aufweisen und die als solche oder im Gemisch mit einem Lösungsmittel verwendet werden;

(d) die Harze unter den Harzen ausgewählt sind, die aus Einheiten $R_3SiO_{1/2}$, $R_2SiO_{2/2}$, $RSiO_{3/2}$ und $SiO_{4/2}$ aufgebaut sind, worin die Gruppe R eine Kohlenwasserstoffgruppe mit 1 bis 16 Kohlenstoffatomen oder eine Phenylgruppe bedeutet;

(e) die organomodifizierten Silicone unter den Siliconen ausgewählt sind, die in ihrer Struktur eine oder mehrere organofunktionelle Gruppen tragen, die über eine Kohlenwasserstoffgruppe gebunden sind.

**21.** Zusammensetzung nach Anspruch 20, **dadurch gekennzeichnet, dass** die einzeln oder im Gemisch verwendeten Silicone unter den folgenden Strukturen ausgewählt sind:

- Polydimethylsiloxan,
- Polydimethylsiloxan/methylvinylsiloxanen,
- Polydimethylsiloxan/diphenylsiloxan,
- Polydimethylsiloxan/phenylmethylsiloxan,
- Polydimethylsiloxan/diphenylsiloxan/methylvinylsiloxan und den folgenden Gemischen:
- Gemischen, die aus einem am Kettenende hydroxylierten Polydimethylsiloxan und einem cyclischen Polydimethylsiloxan gebildet sind,
- Gemischen, die aus einem Polydimethylsiloxangummi und einem cyclischen Silicon gebildet sind, und
- Gemischen von Polydimethylsiloxanen unterschiedlicher Viskositäten.

**22.** Zusammensetzung nach Anspruch 20, **dadurch gekennzeichnet, dass** die organomodifizierten Silicone unter den Polyorganosiloxanen ausgewählt sind, die enthalten:

a) Polyethylenoxy- und/oder Polypropylenoxygruppen,

b) substituierte oder unsubstituierte aminierte Gruppen,
c) Thiolgruppen,
d) alkoxylierte Gruppen,
e) Hydroxyalkylgruppen,
f) Acyloxyalkylgruppen,
g) Carboxyalkylgruppen,
h) 2-Hydroxyalkylsulfonatgruppen,
i) 2-Hydroxyalkylthiosulfonatgruppen,
j) Hydroxyacylaminogruppen.

**23.** Zusammensetzung nach einem der Ansprüche 20 bis 22,
**dadurch gekennzeichnet, dass** die Silicone unter den Polyalkylsiloxanen mit endständigen Trimethylsilylgruppen, Polyalkylsiloxanen mit endständigen Dimethylsilanolgruppen, Polyalkylarylsiloxanen, Gemischen von zwei PDMS, die aus einem Gummi und einem Öl unterschiedlicher Viskositäten gebildet sind, Gemischen von Organosiloxanen und cyclischen Siliconen, Organopolysiloxanharzen ausgewählt sind.

**24.** Zusammensetzung nach Anspruch 13, **dadurch gekennzeichnet, dass** das anionische Polymer ausgewählt ist unter:

- Polymeren, die Carboxyeinheiten aufweisen, die von Mono- oder Dicarbonsäuremonomeren der folgenden Formel abgeleitet sind:

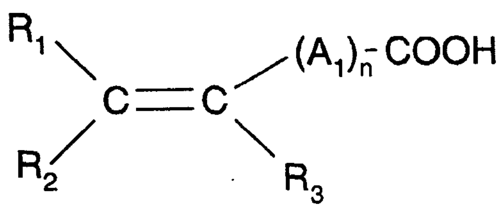

(II)

worin n 0 oder eine ganze Zahl von 1 bis 10 bedeutet, $A_1$ eine Methylengruppe ist, die gegebenenfalls über ein Heteroatom, wie Sauerstoff oder Schwefel, an das Kohlenstoffatom der ungesättigten Gruppe oder, wenn n größer 1 ist, an die benachbarte Methylengruppe gebunden ist, $R_1$ ein Wasserstoffatom, Phenyl oder Benzyl bedeutet, $R_2$ ein Wasserstoffatom, eine niedere Alkylgruppe oder Carboxy ist und $R_3$ ein Wasserstoffatom, eine niedere Alkylgruppe, die Gruppe $-CH_2COOH$, Phenyl oder Benzyl bedeutet;
- Polymeren, die Einheiten aufweisen, die von einer Sulfonsäure abgeleitet sind, wie Vinylsulfonsäure, Styrolsulfonsäure, Acrylamidoalkylsulfonsäure.

**25.** Zusammensetzung nach dem vorhergehenden Anspruch,
**dadurch gekennzeichnet, dass** das anionische Polymer ausgewählt ist unter:

- Copolymeren von Acrylsäure;
- von Crotonsäure abgeleiteten Copolymeren;
- Polymeren, die von Maleinsäure oder Maleinsäureanhydrid, Fumarsäure oder Itaconsäure und Vinylestern, Vinylethern, Vinylhalogeniden, Phenylvinylderivaten, Acrylsäure und ihren Estern abgeleitet sind;
- Copolymeren von Methacrylsäure und Methylmethacrylat;
- dem Copolymer von Methacrylsäure und Ethylacrylat;
- dem Vinylacetat/Crotonsäure-Copolymer;
- dem Vinylacetat/Crotonsäure/Polyethylenglycol-Terpolymer.

**26.** Zusammensetzung nach Anspruch 13, **dadurch gekennzeichnet, dass** das amphotere Polymer unter den Polymeren ausgewählt ist, die Einheiten aufweisen, abgeleitet von:

a) mindestens einem Monomer, das unter den Acrylamiden oder Methacrylamiden ausgewählt ist, die am Stickstoff mit einer Alkylgruppe substituiert sind;

b) mindestens einem Säurecomonomer, das eine oder mehrere reaktive Carboxygruppen aufweist; und
c) mindestens einem basischen Comonomer, wie Estern von Acrylsäure und Methacrylsäure mit primären, sekundären, tertiären und quartären Aminosubstituenten, und dem Quaternisierungsprodukt von Dimethylaminoethylmethacrylat mit Dimethylsulfat oder Diethylsulfat.

**27.** Zusammensetzung nach Anspruch 13, **dadurch gekennzeichnet, dass** das nichtionische Polymer ausgewählt ist unter:

- Polyalkyloxazolinen;
- Homopolymeren von Vinylacetat;
- Copolymeren von Vinylacetat und einem Acrylester;
- Copolymeren von Vinylacetat und Ethylen;
- Copolymeren von Vinylacetat und einem Maleinsäureester;
- Copolymeren von Polyethylen und Maleinsäureanhydrid;
- Homopolymeren von Alkylacrylaten und Homopolymeren von Alkylmethacrylaten;
- Copolymeren von Acrylestern;
- Copolymeren von Acrylnitril und einem nichtionischen Monomer; und
- Copolymeren von Alkylacrylat und Urethan.

**28.** Zusammensetzung nach Anspruch 13, **dadurch gekennzeichnet, dass** die kationischen Polymere unter den Polymeren ausgewählt sind, die entweder als Teil der Polymerhauptkette oder an einem direkt daran gebunden seitlichen Substituenten Einheiten mit primären, sekundären, tertiären und/oder quartären Aminogruppen aufweisen.

**29.** Zusammensetzung nach Anspruch 28, **dadurch gekennzeichnet, dass** das kationische Polymer unter den kationischen Cyclopolymeren, kationischen Polysacchariden, quartären Polymeren von Vinylpyrrolidon und Vinylimidazol und deren Gemischen ausgewählt ist.

**30.** Zusammensetzung nach Anspruch 29, **dadurch gekennzeichnet, dass** das Cyclopolymer unter den Homopolymeren von Diallyldimethylammoniumchlorid und den Copolymeren von Diallyldimethylammoniumchlorid und Acrylamid ausgewählt ist.

**31.** Zusammensetzung nach Anspruch 29, **dadurch gekennzeichnet, dass** die kationischen Polysaccharide unter den Hydroxyethylcellulosen ausgewählt sind, die mit einem mit einer Trimethylammoniumgruppe substituierten Epoxid reagiert haben.

**32.** Zusammensetzung nach Anspruch 29, **dadurch gekennzeichnet, dass** die kationischen Polysaccharide unter den mit einem 2,3-Epoxypropyltrimethylammoniumsalz modifizierten Guargummen ausgewählt sind.

**33.** Zusammensetzung nach Anspruch 13, **dadurch gekennzeichnet, dass** die kationischen grenzflächenaktiven Stoffe ausgewählt sind unter:

(A) quartären Ammoniumsalzen der folgenden allgemeinen Formel (XXIII):

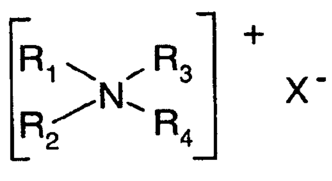

(XXIII)

worin X ein Anion ist, das unter den Halogeniden, Alkyl($C_{2-6}$)sulfaten, Phosphaten, Alkyl- oder Alkylarylsulfonaten und von einer organischen Säure abgeleiteten Anionen ausgewählt ist, und

i) die Gruppen $R_1$ bis $R_3$, die gleich oder verschieden sein können, eine geradkettige oder verzweigte aliphatische Gruppen mit 1 bis 4 Kohlenstoffatomen oder eine aromatische Gruppe bedeuten, wobei die aliphatischen Gruppen Heteroatome enthalten können; die Gruppe $R_4$ eine geradkettige oder verzweigte Alkylgruppe mit 16 bis 30 Kohlenstoffatomen ist;

ii) die Gruppen $R_1$ und $R_2$, die gleich oder verschieden sein können, eine geradkettige oder verzweigte aliphatische Gruppen mit 1 bis 4 Kohlenstoffatomen oder eine aromatische Gruppe bedeuten, wobei die aliphatischen Gruppen Heteroatome enthalten können;
die Gruppen $R_3$ und $R_4$, die gleich oder verschieden sein können, eine geradkettige oder verzweigte Alkylgruppe mit 12 bis 30 Kohlenstoffatomen bedeuten, wobei die Gruppe mindestens eine Ester- oder Amidfunktion aufweist;

(B) quartären Ammoniumsalzen von Imidazolinium;
(C) quartären Diammoniumsalzen der Formel (XXV):

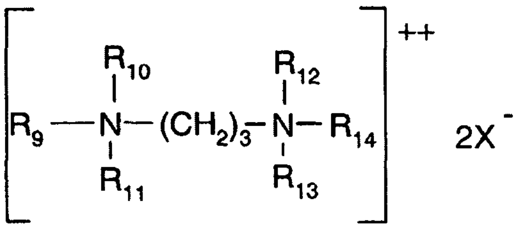

(XXV)

worin die Gruppe $R_9$ eine aliphatische Gruppe mit etwa 16 bis 30 Kohlenstoffatomen bedeutet, die Gruppen $R_{10}$, $R_{11}$, $R_{12}$, $R_{13}$ und $R_{14}$, die gleich oder verschieden sind, unter Wasserstoff oder einer Alkylgruppe mit 1 bis 4 Kohlenstoffatomen ausgewählt sind, und X ein Anion ist, das unter den Halogeniden, Acetaten, Phosphaten, Nitraten und Methylsulfaten ausgewählt ist;
(D) quartären Ammoniumsalzen, die mindestens eine Esterfunktion der folgenden Formel (XXVI) enthalten:

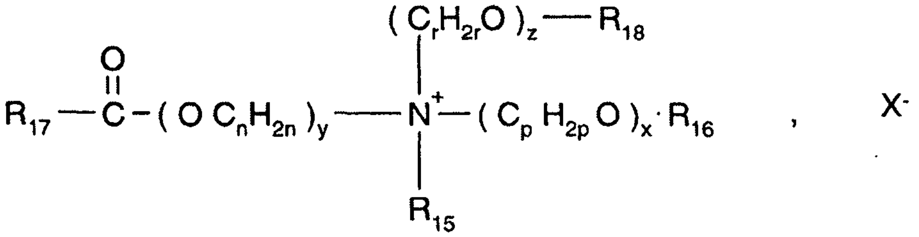

(XXVI)

in der Formel:

- $R_{15}$ ist unter den $C_{1-6}$-Alkylgruppen und den Hydroxyalkyl- oder Dihydroxyalkylgruppen mit 1 bis 6 Kohlenstoffatomen ausgewählt;
- $R_{16}$ ist ausgewählt unter:
- der Gruppe

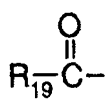

- den Gruppen $R_{20}$ auf Kohlenwasserstoffbasis mit 1 bis 22 Kohlenstoffatomen, die geradkettig oder verzweigt, gesättigt oder ungesättigt sind,
- dem Wasserstoffatom,
- $R_{18}$ ist ausgewählt unter:
- der Gruppe

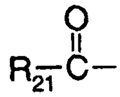

- den Gruppen $R_{22}$ auf Kohlenwasserstoffbasis mit 1 bis 6 Kohlenstoffatomen, die geradkettig oder verzweigt, gesättigt oder ungesättigt sind,
- dem Wasserstoffatom,
- $R_{17}$, $R_{19}$ und $R_{21}$, die identisch oder voneinander verschieden sind, sind unter den geradkettigen oder verzweigten, gesättigten oder ungesättigten $C_{7-21}$-Kohlenwasserstoffgruppen ausgewählt;
- n, p und r, die gleich oder verschieden sind, sind ganze Zahlen von 2 bis 6;
- y ist eine ganze Zahl im Bereich von 1 bis 10;
- x und z, die gleich oder verschieden sind, bedeuten 0 oder eine ganze Zahl von 1 bis 10;
- $X^-$ ist ein einfaches oder komplexes, organisches oder anorganisches Anion;

mit der Maßgabe, dass die Summe x + y + z im Bereich von 1 bis 15 liegt, dass $R_{16}$ $R_{20}$ bedeutet, wenn x 0 ist, und dass $R_{18}$ $R_{22}$ bedeutet, wenn z 0 ist.

**34.** Zusammensetzung nach einem der vorhergehenden Ansprüche, **dadurch gekennzeichnet, dass** das Konditioniermittel in einer Konzentration von 0,001 bis 20 Gew.-%, bezogen auf das Gesamtgewicht der Zusammensetzung, vorliegt.

**35.** Zusammensetzung nach einem der vorhergehenden Ansprüche, **dadurch gekennzeichnet, dass** sie mindestens ein kationisches Polymer und/oder mindestens ein Silicon enthält.

**36.** Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren oder kationischen grenzflächenaktiven Stoffen und deren Gemischen ausgewählt ist.

**37.** Zusammensetzung nach Anspruch 36, **dadurch gekennzeichnet, dass** der oder die grenzflächenaktiven Stoffe in einer Konzentration im Bereich von 0,1 bis 60 Gew.-%, bezogen auf das Gesamtgewicht der Zusammensetzung, enthalten sind.

**38.** Zusammensetzung nach einem der Ansprüche 1 bis 37,
**dadurch gekennzeichnet, dass** die Zusammensetzung ferner mindestens einen Zusatzstoff enthält, der unter den Verdickungsmitteln, Wirkstoffen gegen Schuppen oder gegen Seborrhöe, Parfums, Perlglanzstoffen, Hydroxysäuren, Elektrolyten, Konservierungsmitteln, siliconierten oder nicht siliconierten Sonnenschutzfiltern, Vitaminen, Provitaminen, anionischen oder nichtionischen Polymeren, Proteinen, Proteinhydrolysaten, 18-Methyleicosansäure, Panthenol sowie den Gemischen dieser verschiedenen Verbindungen ausgewählt ist.

**39.** Zusammensetzung nach einem der vorhergehenden Ansprüche, **dadurch gekennzeichnet, dass** sie als Haarwaschmittel, Produkt, das nach der Haarwäsche angewandt wird, Zusammensetzung für eine dauerhafte Verformung, Entkräuselung, Färbung oder Entfärbung der Haare, Zusammensetzung, die ausgespült wird und zwischen den beiden Schritten einer dauerhaften Verformung oder einer Entkräuselung aufgebracht wird, oder reinigende Zusammensetzung für den Körper vorliegt.

**40.** Verwendung einer Zusammensetzung, wie sie in einem der vorhergehenden Ansprüche definiert ist, für die Reinigung oder für die Pflege von Keratinsubstanzen.

**41.** Verfahren zur Behandlung von Keratinsubstanzen wie Haaren, **dadurch gekennzeichnet, dass** es darin besteht, eine kosmetische Zusammensetzung nach einem der Ansprüche 1 bis 39 auf die Keratinsubstanzen aufzutragen und dann gegebenenfalls mit Wasser zu spülen.

**42.** Verwendung einer Zusammensetzung nach einem der Ansprüche 1 bis 39 zum Konditionieren von Keratinsubstanzen.

**43.** Verwendung einer Zusammensetzung nach einem der Ansprüche 1 bis 39, um die Lockerheit, die Weichheit, den

Glanz und/oder die Kämmbarkeit zu verbessern und das Frisieren von Keratinsubstanzen zu erleichtern.

**44.** Verwendung einer Zusammensetzung nach einem der Ansprüche 1 bis 39, um die Remanenz der Konditionierwirkung bei der Haarwäsche zu verbessern.

**45.** Verwendung mindestens eines aminierten Silicons, wie es in einem der Ansprüche 1 bis 12 definiert ist, um die Remanenz der Konditionierwirkung einer kosmetischen Zusammensetzung bei der Haarwäsche zu verbessern.

**46.** Verfahren, um die Remanenz der Konditionierwirkung einer kosmetischen Zusammensetzung bei der Haarwäsche zu verbessern, das darin besteht, ein aminiertes Silicon, wie es in einem der Ansprüche 1 bis 12 definiert ist, in eine kosmetische Zusammensetzung einzuarbeiten.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 4601902 A
- DE 4424530
- DE 4424533
- DE 4402929
- DE 4420736
- WO 9523807 A
- WO 9407844 A
- EP 0646572 A
- WO 9516665 A
- FR 2673179
- EP 0227994 A
- WO 9424097 A
- WO 9410131 A
- EP 486135 A
- WO 9311103 A
- FR 8516334 A
- US 4957732 A
- EP 186507 A
- EP 342834 A
- EP 412704 A
- EP 412707 A
- EP 640105 A
- WO 9500578 A
- EP 582152 A
- WO 9323009 A
- US 4693935 A
- US 4728571 A
- US 4972037 A
- FR 1222944
- DE 2330956
- FR 1580545
- FR 2265782
- FR 2265781
- FR 1564110
- FR 2439798
- US 2047398 A
- US 2723248 A
- US 2102113 A
- GB 839805 A
- FR 2350384
- FR 2357241
- FR 2198719
- US 4128631 A
- FR 1400366
- EP 0751162 A
- EP 0637600 A
- FR 2743297
- EP 0648485 A
- EP 0656021 A
- WO 9403510 A
- EP 0619111 A
- EP 0337354 A
- FR 2270846 A
- FR 2383660 A
- FR 2598611 A
- FR 2470596 A
- FR 2519863 A
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- FR 2080759
- FR 2190406
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- US 2528378 A
- US 2781354 A

**Littérature non-brevet citée dans la description**


- **P.D. Dorgan.** *Drug and Cosmetic Industry,* Décembre 1983, 30-33
- **TODD ; BYERS.** Volatile Silicone fluids for cosmetics. *Cosmetics and toiletries,* Janvier 1976, vol. 91, 27-32
- **M.R. PORTER.** Handbook of Surfactants. éditions Blackie & Son, 1991, 116-178